# EUROPÄISCHE PATENTSCHRIFT

(11) **EP 1 984 274 B1**
(45) Veröffentlichungstag und Bekanntmachung des Hinweises auf die Patenterteilung: **08.09.2010**
(21) Anmeldenummer: 06829595.5
(22) Anmeldetag: 14.12.2006
(51) Int. Cl.: B65D 51/28

(54) **ZWEITEILIGER VERSCHLUSS**
TWO-PART CLOSURE
FERMETURE EN DEUX PIECES

(30) Priorität: 13.02.2006 DE 102006006771
(43) Veröffentlichungstag der Anmeldung: 29.10.2008
(73) Patentinhaber: Henkel AG & Co. KGaA, 40589 Düsseldorf (DE)
(72) Erfinder: NACHTSHEIM, Markus, 40764 Langenfeld (DE); MÜHLHAUSEN, Hans-Georg, 40597 Düsseldorf (DE)
(86) Internationale Anmeldenummer: PCT/EP2006/012038
(87) Internationale Veröffentlichungsnummer: WO 2007/093209

(56) Entgegenhaltungen:
- EP-A- 0 119 519
- WO-A-2004/084660
- DE-A1- 3 804 668
- GB-A- 2 314 065

## Beschreibung

Die Erfindung betrifft einen Verschluss mit den Merkmalen gemäß dem Oberbegriff des Anspruchs 1 sowie eine Verpackung gemäß dem Oberbegriff des Anspruchs 20. Es handelt sich dabei um einen zweiteiligen Verschluss, insbesondere einen Dosierverschluss für fließ- oder schüttfähige Produkte, mit einer abnehmbaren, eine Aktivsubstanz beinhaltenden Kammer.

### Stand der Technik

Es besteht seit langem ein Bedürfnis danach, Produkte kundenspezifisch individualisieren zu können oder durch den Kunden in einem gewissen Maße individualisieren zu lassen. Ein besonders geeignetes Medium zur Individualisierung eines Produktes stellt seine Verpackung dar, da diese die unmittelbare Schnittstelle zwischen Verbraucher und Produkt ausbildet.

Daher ist es wünschenswert, Mittel zur Individualisierung oder weiteren Funktionalisierung eines Produktes unmittelbar mit der Verpackung eines Produktes zu koppeln.

So besteht beispielsweise ein Bedürfnis danach, dass eine Produktverpackung derart ausgestaltet ist, das der Duft des in der Verpackung enthaltenen Produkts olfaktometrisch wahrnehmbar ist. Verpackungen werden daher zunehmend dahin gehend zusätzlich funktionalisiert, dass an der Verpackung duftemittierende Mittel angeordnet sind, die einen bestimmten Duft in die Umgebung der Verpackung abgeben und so die zusätzliche Funktion eines Raumbedufters übernehmen.

Aus WO2004/084660 ist bekannt, duftstoffemittierende Substanzen in einer Verschlusskappe eines Behälters anzuordnen. Hierbei sind in einer Kammer des Behälterverschlusses Duftstoff emittierende Substanzen angeordnet, wobei diese Kammer mit einer Folie versiegelt ist, die zum Gebrauch der Raumbedufterkappe entfernt werden kann, so dass Duftstoff aus der Verschlusskappe an die Umgebung abgegeben werden kann. Nachteilig an dieser Lösung ist, dass die Funktion des Raumbedufters an den Verschluss bzw. an den Behälter gekoppelt ist. So ist es nicht möglich, die duftstoffemittierende Verschlusskappe unabhängig und separat vom Behälter zu positionieren, ohne den Behälter offen zurück zu lassen. Weitere, gattungsgemäße Verschlosskappen sind aus GB-A-2 314 065 und EP-A-0 119 519 bekannt.

Aufgabe der Erfindung ist es daher eine kostengünstig zu fertigende eine Aktivsubstanz beinhaltende Verschlusskappe sowie eine Verpackung damit zu entwickeln, die die vorgenannten Nachteile überwindet.

Diese Aufgabe wird gemäß den Merkmalen des Anspruchs 1 bzw. 20 gelöst.

Durch die vom erfindungsgemäßen Verschluss abnehmbare, mit Aktivsubstanz insbesondere Duftstoff, befüllte Kammer kann der Verschluss sowohl am "Point of Sale" als Geruchsindikator dienen, als auch durch den Benutzer an der oder getrennt von der Verschlusskappe als Lufterfrischer verwendet werden. Ferner ist es möglich, den Verschluss mit Indikatorsubstanzen zu befüllen und als Indikator für physikalische/ chemische Parameter, beispielsweise für den pH-Wert einer Reinigungslösung oder die Temperatur von Wischwasser zu benutzen. Des Weiteren kann die Kammer eine Aktivsubstanz zur Verstärkung einer Wirkung bzw. Funktion der im Behälter befindlichen Zubereitung beinhalten, beispielsweise einen Schaumbildner zur Verbesserung des Dufteindrucks oder ein Bleichmittel zur Verstärkung der Reinigungsleistung einer Zubereitung, der dann mittels der abnehmbaren Kammer durch den Anwender einfach dosiert werden kann.

Ferner ist die erfindungsgemäße Lösung leicht an bestehende Verschlüsse adaptierbar.

Die Verschlusskappe kann als Schraubkappe, Push-Pull-Verschluss, Flipp-Top-Verschluss, Klappverschluss, Hebelverschluss, Stopfen, Dosierverschluss oder dergleichen ausgeführt sein.

Die Verschlusskappe kann weitere Dosier- und Dosierhilfsmittel und Entnahmehilfen wie beispielsweise Triggerpumpen, Dosierpumpen, Dosierspender, Aerosolventile, Ausgießer, Sprühkappen, Sprühköpfe, Zerstäuber oder Tropfer umfassen.

In einer bevorzugten Ausführungsform der Erfindung weist die Verschlusskappe einen Überwurfring auf, an dem ein innenliegendes Schraubengewinde angeordnet ist, dass auf ein korrospondierendes außenliegendes Schraubgewinde eines Behälters aufgeschraubt werden kann und den Behälter somit verschließt.

Die Kammer kann form- und/oder stoff- und/oder kraftschlüssig an der Verschlusskappe fixiert sein.

Die Kammer und die Verschlusskappe können insbesondere so ausgebildet sein und/oder zusätzliche Verbindungsmittel umfassen, so dass die Kammer und die Verschlusskappe formschlüssig beispielsweise durch einen Schnappverschluss, Rastverschluss, Prellverschluss, Schraubverschluss, Bajonettverschluss, Klemmverschluss, Quetschverschluss oder Druckknopfverschluss aneinander fixierbar sind.

In einer weiteren Ausgestaltungsform der Erfindung kann die Kammer auch stoffschlüssig an der Verschlusskappe angeordnet sein. Die stoffschlüssige Verbindung kann derart ausgestaltet sein, dass beim Lösen der Kammer von der Verschlusskappe die stoffschlüssige Verbindung zerstört wird und nicht wieder herstellbar ist, so dass die Kammer nach der Abnahme von der Verschlusskappe nicht mehr durch die ursprüngliche stoffschlüssige Verbindung an der Verschlusskappe haftet. Die stoffschlüssige Verbindung kann jedoch auch derart ausgebildet sein, dass ein wiederholtes Lösen und Haften der Kammer auf der Verschlusskappe ermöglicht wird.

Die stoffschlüssige Verbindung kann beispielsweise aus der Gruppe der Klebeverbindungen, Schweißverbindungen oder Siegelverbindungen ausgewählt sein.

Ferner ist es möglich die lösbare Verbindung zwischen der Kammer und der Verschlusskappe durch Kraftschluss herzustellen. Dies kann beispielsweise durch eine Pressverbindung, Reibverbindung, Klemmverbindung, Quetschverbindung, Schrumpfverbindung, Keilverbindung, Schraubverbindung, Stiftverbindung oder dergleichen realisiert sein.

In einer besonders bevorzugten Ausführungsform der Erfindung ist eine aus der Verschlusskappe herausragende domartige Erhöhung an der Verschlusskappe ausgebildet, auf der die Kammer kraftschlüssig durch eine lösbare Pressverbindung aufsteckbar ist.

Gemäß einer bevorzugten Ausführungsform der Erfindung ist die Verbindung zwischen der Kammer und der Verschlusskappe derart ausgestaltet, dass sie durch eine Zugkraft von größer als 1 N entlang der Längsachse von der Verschlusskappe lösbar ist. Dies gewährleistet einen hinreichenden Halt der Kammer auf der Verschlusskappe und verhindert ein unbeabsichtigtes Lösen der Kappe z.B. während des Transports.

Bevorzugter Weise weist die Verschlusskappe ein becherartig ausgeformtes Dosiervolumen auf. Besonders bevorzugt beträgt das Dosiervolumen zwischen 5 ml und 100 ml, insbesondere zwischen 10 ml und 40 ml.

In einer weiteren Ausführungsform der Erfindung ist die Kammer zweiteilig ausgeformt und umfasst ein becherförmiges Element und eine Verschlussplatte, die die Öffnung des becherförmigen Elements verschließt. Der Verschluss kann durch Stoffschluss, Formschluss oder Kraftschluss hergestellt sein. Gemäß einer besonders bevorzugten Ausführungsform der Erfindung ist der Verschluss der Becheröffnung flüssigkeits- oder im Wesentlichen gasdichtig ausgebildet.

Besonders vorteilhaft ist es, eine umlaufende Nut auf der Mantelfläche des becherförmigen Elements oder der Verschlussplatte vorzusehen, die mit einer korrespondierenden Nase der Verschlussplatte bzw. des becherförmigen Elements derart zusammenwirkt, dass eine Rastverbindung zwischen Verschlussplatte und Becherelement ausgebildet wird.

Ferner kann die Kammer außenseitig ein Befestigungselement aufweisen, das zur Fixierung der Kammer an Strukturen außerhalb des Dosierverschlusses geeignet ist. Beispielsweise kann das Befestigungselement als versiegelter Klebestreifen ausgebildet sein, wobei die Versiegelung nach Abnahme der Kammer von der Verschlusskappe abgelöst wird und die Kammer somit beispielsweise auf Oberflächen in Schränken oder in oder auf Mülleimern aufklebbar ist.

Die Kammer kann eine oder mehrere Aktivsubstanzen in fester und/oder flüssiger und/oder gelartiger und/oder gasförmiger Form beinhalten.

Die Aktivsubstanzen können auch an oder in Trägermaterialen gebunden oder gelöst sein. Die Trägermaterialien können einen festen und/oder flüssigen und/oder gelartigen und/oder gasförmigen Aggregatszustand aufweisen.

Der Inhalt der Kammer kann eine oder mehrere gleiche oder unterschiedliche Aktivsubstanzen aus der Gruppe der Duftstoffe, Bleichmittel, Reinigungssubstanzen, Lösemittel, Tenside, Farbstoffe, Enzyme, hygroskopische Substanzen, Flammhemmer, Härter, Verlaufsmittel, Netzmittel, Dispergiermittel, Schaumbildner, Entschäumer, Wachse, Silikone, Entlüfter, Korrosionsschutzmittel, Biozide, Insektizide, Wasserenthärter, Konservierungsmittel, Adsorptionsmittel, Absorptionsmittel, Abrasivstoffe, Emulgatoren, Stabilisatoren, Vitamine, Mineralien und dergleichen umfassen.

Die Aktivsubstanzen können des Weiteren auch Substanzen oder Substanzgemische zur Hydrophilisierung von Oberflächen umfassen.

Ferner ist es möglich, Indikatorsubstanzen zur Bestimmung der Wasserhärte, Keimbelastung, Temperatur, Feuchtigkeit usw. in der Kammer zu bevorraten.

Es ist insbesondere von Vorteil einen Schaumbildner in Verbindung mit einem Duftstoff zu verwenden. So kann beispielsweise der jeweils der Duftstoff, der Schaumbildner oder beide in der Kammer des Verschlusses bevorratet sein. Es ist somit beispielsweise denkbar eine mit Duftstoff versetzte Zubereitung in einem Behälter zu füllen und in der Kammer des Verschlusses den Schaumbildner anzuordnen. Der Schaumbildner kann dann zur unmittelbaren Anwendung der im Behälter befindlichen Zubereitung zudosiert werden, indem die Kammer als Ganzes zugesetzt wird, beispielsweise indem sie in einen mit Zubreitung gefüllten Eimer gegeben wird, oder durch Abgabe von Schaumbildner, beispielsweise als streufähige Zubereitung, aus der Kammer.

Durch die Schaumbildung wird die Oberfläche der Duftstoff enthaltenden Zubereitung erhöht, so dass es eine verbesserte Duftabgabe und Duftwahrnehmung durch den Benutzer bewirkt wird.

Gemäß einer bevorzugten Ausführungsform der Erfindung, sind die wirkaktiven Substanzen an oder in einem polymeren Trägermaterial gebunden. Besonders bevorzugt werden Duftstoffe in oder an einem polymeren Trägermaterial gebunden.

Zur Freisetzung der Aktivsubstanzen aus der Kammer in die Umgebung weist die Kammer mindestens eine Öffnung auf. Die Öffnung ist bevorzugt so ausgestaltet, dass das Trägermaterial die Öffnung nicht passieren kann.

Gemäß einer alternativen Ausführungsform, kann die Öffnung auch so ausgestaltet sein, dass sie durch die in der Kammer befindlichen Aktivsubstanz passiert werden kann. Beispielsweise kann die Aktivsubstanz als streufähige oder fließfähige Zubereitung ausgebildet sein. Insbesondere kann die Kammer derart gestaltet sein, dass sie wie ein Salzstreuer eine streufähige Zubereitung durch die entsprechende Öffnung hindurch an die Umgebung abgeben kann.

In einer weiteren bevorzugten Ausgestaltung der Erfindung weist der Verschluss ein Verschlusselement auf, welches in einer Verschlussposition die Abgabe von Aktivsubstanz aus der Kammer durch die Öffnungen an die Umgebung zumindest reduziert und in einer geöffneten Position die Abgabe von Aktivsubstanz aus der Kammer ermöglicht.

Beispielsweise kann das Verschlusselement als Abreißdeckel, Aufsatzdeckel, Falzdeckel, Rillendeckel, Scharnierdeckel, Schiebedeckel, Schnappdeckel, Schraubdeckel, Stülpdeckel, Stopfen ausgeformt sein.

Besonders bevorzugt ist es, das Verschlusselement derart auszuführen, dass ein wiederholtes Freigeben und Verschließen der Öffnungen ermöglicht wird.

### Polymeres Trägermaterial

Für die duftstoffhaltigen Partikel eignen sich generell alle Polymere oder Polymergemische, welche die oben genannten Kriterien bezüglich der Schmelz- oder Erweichungstemperatur erfüllen. Im Rahmen der vorliegenden Anmeldung bevorzugte Duftabgabesysteme sind **dadurch gekennzeichnet, daß** das polymere Trägermaterial mindestens eine Substanz aus der Gruppe umfassend EthylenNinylacetat- Copolymere, Polyethylen niederer oder hoher Dichte (LDPE, HDPE) oder Gemische derselben, Polypropylen, Polyethylen/Polypropylen-Copolymere, Polyether/Polyamid-Blockcopolymere, Styrol/Butadien-(Block-)Copolymere, Styrol/Isopren-(Block-)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat, Polyurethane, Polyvinylalkohole enthält.

Polyethylen (PE) ist eine Sammelbezeichnung für zu den Polyolefinen gehörende Polymere mit Gruppierungen des Typs

CH₂-CH₂

als charakteristische Grundeinheit der Polymerkette. Polyethylene werden in der Regel durch Polymerisation von Ethylen nach zwei grundsätzlich unterschiedlichen Methoden, dem Hochdruck- und dem Niederdruck-Verfahren hergestellt. Die resultierenden Produkte werden entsprechend häufig als Hochdruck-Polyethylene bzw. Niederdruck-Polyethylene bezeichnet; sie unterscheiden sich hauptsächlich hinsichtlich ihres Verzweigungsgrades und damit verbunden in ihrem Kristallinitätsgrad und ihrer Dichte. Beide Verfahren können als Lösungspolymerisation, Emulsionspolymerisation oder Gasphasenpolymerisation durchgeführt werden.

Beim Hochdruck-Verfahren fallen verzweigte Polyethylene mit niedriger Dichte (ca. 0,915-0,935 g/cm³) und Kristallinitätsgraden von ca. 40-50% an, die man als LDPE-Typen (low density polyethylene) bezeichnet. Produkte mit höherer Molmasse und dadurch bedingter verbesserter Festigkeit und Streckbarkeit tragen die Kurzbezeichnung HMW-LDPE (HMW=high molecular weight). Durch Copolymerisation des Ethylens mit längerkettigen Olefinen, insbesondere mit Buten und Octen, kann der ausgeprägte Verzweigungsgrad der im Hochdruck-Verfahren hergestellten Polyethylene reduziert werden; die Copolymere haben das Kurzzeichen LLD-PE (linear low density polyethylene).

Die Makromoleküle der Polyethylene aus Niederdruck-Verfahren sind weitgehend linear und unverzweigt. Diese Polyethylene, Kurzzeichen HDPE (von E high density polyethylene) haben Kristallinitätsgrade von 60-80% und eine Dichte von ca. 0,94-0,965 g/cm3. Sie werden als Produkte mit hoher bzw. ultrahoher Molmasse (ca. 200 000-5 000 000 g/mol bzw. 3 000 000-6 000 000 g/mol) unter der Kurzbezeichnung HD-HMW-PE bzw. UHMW-HD-PE angeboten. Auch Produkte mit mittlerer Dichte (MDPE) aus Mischungen von Polyethylenen niedriger und hoher Dichte sind kommerziell erhältlich. Lineare Polyethylene mit Dichten <0,918 g/cm3 (VLD-PE, von E very low density polyethylene) gewinnen nur langsam Marktbedeutung.

Polyethylene haben eine sehr geringe Wasserdampfdurchlässigkeit, die Diffusion von Gasen sowie von Aromastoffen und etherischen Substanzen durch Polyethylene ist relativ hoch. Die mechanischen Eigenschaften sind stark abhängig von Molekülgröße und -struktur der Polyethylene. Generell steigen Kristallinitätsgrad und Dichte von Polyethylene mit abnehmendem Verzweigungsgrad und mit Verkürzung der Seitenketten an. Mit der Dichte steigen Schubmodul, Härte, Streckgrenze und Schmelzbereich; es nehmen ab Schockfestigkeit, Transparenz, Quellbarkeit und Löslichkeit. Bei gleicher Dichte nehmen mit steigender Molmasse der Polyethylene Reißfestigkeit, Dehnung, Schockfestigkeit, Schlagzähigkeit und Dauerstandfestigkeit zu. Je nach Arbeitsweise bei der Polymerisation kann man Produkte mit Paraffinwachsähnlichen Eigenschaften (MR um 2000) und Produkte mit höchster Zähigkeit (MR über 1 Mio.) erhalten.

Die Verarbeitung der Polyethylen-Typen kann nach allen für Thermoplaste üblichen Methoden erfolgen.

Polypropylen (PP) ist die Bezeichnung für thermoplastische Polymere des Propylens mit der allg. Formel:

-(CH₂-CH[CH3])ₙ-

Basis für die Polypropylen-Herstellung war die Entwicklung des Verfahrens zur stereospezifischen Polymerisation von Propylen in der Gasphase oder in Suspension durch Natta. Diese wird mit Ziegler-Natta-Katalysatoren, in zunehmendem Maße aber auch durch Metallocen-Katalysatoren initiiert und führt entweder zu hochkristallinen isotaktischen oder zu weniger kristallinen syndiotaktischen bzw. zu amorphen ataktischen Polypropylenen.

Polypropylen zeichnet sich durch hohe Härte, Rückstellfähigkeit, Steifheit und Wärmebeständigkeit aus. Kurzfristiges Erwärmen von Gegenständen aus Polypropylen ist sogar bis 140 °C möglich. Bei Temperaturen unter 0 °C tritt eine gewisse Versprödung der Polypropylene ein, die jedoch durch Copolymerisation des Propylens mit Ethylen (EPM, EPDM) zu wesentlich tieferen Temperaturbereichen verschoben werden kann. Allgemein läßt sich die Schlagzähigkeit von Polypropylen durch Modifikation mit Elastomeren verbessern. Die Chemikalienbeständigkeit ist wie bei allen Polyolefinen gut. Eine Verbesserung der mechanische Eigenschaften der Polypropylene erreicht man durch Verstärkung mit Talkum, Kreide, Holzmehl oder Glasfasern. Polypropylene sind in noch stärkerem Maße als PE oxidations- und lichtempfindlich, weshalb der Zusatz von Stabilisatoren (Antioxidantien, Lichtschutzmittel, UV-Absorber) erforderlich ist.

Polyether ist eine auf dem Gebiet der Makromolekularen Chemie übergreifende Bezeichnung für Polymere, deren organische Wiederholungseinheiten durch Ether-Funktionalitäten (C-O-C) zusammengehalten werden. Nach dieser Definition gehört eine Vielzahl strukturell sehr unterschiedlicher Polymerer zu den Polyethern, z. B. die Polyalkylenglykole (Polyethylenglykole, Polypropylenglykole und Polyepichlorhydrine) als Polymere von 1,2-Epoxiden, Epoxidharze, Polytetrahydrofurane (Polytetramethylenglykole), Polyoxetane, Polyphenylenether (s. Polyarylether) oder Polyetheretherketone (s. Polyetherketone). Nicht zu den Polyethern werden Polymere mit seitenständigen Ether-Gruppen gerechnet, wie u. a. die Celluloseether, Stärkeether und Vinylether-Polymere.

Zur Gruppe der Polyether zählen weiterhin auch funktionalisierte Polyether, d. h. Verbindungen mit einem Polyether-Gerüst, die an ihren Hauptketten seitlich angeheftet noch andere funktionelle Gruppen tragen wie z. B. Carboxy-, Epoxy-, Allyl- oder Amino-Gruppen usw. Vielseitig verwendbar sind Block-Copolymere von Polyethern und Polyamiden (sog. Polyetheramide oder Polyether-Blockamide, PEBA).

Als Polyamide (PA) werden Polymere bezeichnet, deren Grundbausteine durch Amid-Bindungen (-NH-CO-) zusammengehalten werden. Natürlich vorkommende Polyamide sind Peptide, Polypeptide und Proteine (Beisp.: Eiweiß, Wolle, Seide). Die synthetischen Polyamide sind bis auf wenige Ausnahmen thermoplastische, kettenförmige Polymere, von denen einige große technische Bedeutung als Synthesefasern und Werkstoffe erlangt haben. Nach dem chemischen Aufbau lassen sich die sogenannte Homo-Polyamide in zwei Gruppen einteilen, die Aminocarbonsäure-Typen (AS) und die Diamin-Dicarbonsäure-Typen (AA-SS; dabei bezeichnen A Amino-Gruppen und S Carboxy-Gruppen). Erstere werden aus nur einem einzigen Monomeren durch z. B. Polykondensation einer w-Aminocarbonsäure (1) (Polyaminosäuren) oder durch ringöffnende Polymerisation cyclischer Amide (Lactame) (2) hergestellt.

Neben den Homopolyamiden haben auch einige Co-Polyamide Bedeutung erlangt. Üblich ist bei diesen eine qualitative und quantitative Angabe der Zusammensetzung z. B. PA 66/6 (80:20) für aus 1,6-Hexandiamin, Adipinsäure und ε-Caprolactam im Molverhältnis 80:80:20 hergestellte Polyamide. Wegen ihrer besonderen Eigenschaften werden Polyamide, die ausschließlich aromatische Reste enthalten (z. B. solche aus p-Phenylendiamin und Terephthalsäure), unter der Gattungsbez. Aramide oder Polyaramide zusammengefaßt (Beisp.: Nomex®).

Die am häufigsten verwendeten Polyamid-Typen (v. a. PA 6 und PA 66) bestehen aus unverzweigten Ketten mit mittleren Molmassen von 15 000 bis 50 000 g/mol. Sie sind im festen Zustand teilkristallin und haben Kristallisationsgrade von 30-60%. Eine Ausnahme bilden Polyamide aus Bausteinen mit Seitenketten oder Co-Polyamide aus stark unterschiedlichen Komponenten, die weitgehend amorph sind. Im Gegensatz zu den im allgemeinen milchig-opaken, teilkristallinen Polyamiden sind diese fast glasklar. Die Erweichungstemperatur der gebräuchlichsten Homo-Polyamide liegen zwischen 200 und 260 °C (PA 6: 215-220 °C, PA 66: 255-260 °C).

Polyester ist die Sammelbezeichnung für Polymere, deren Grundbausteine durch Ester-Bindungen (-CO-O-) zusammengehalten werden. Nach ihrem chemischen Aufbau lassen sich die sogenannte Homopolyester in zwei Gruppen einteilen, die Hydroxycarbonsäure-Typen (AB-Polyester) und die Dihydroxy-Dicarbonsäure-Typen (AA-BB-Polyester). Erstere werden aus nur einem einzigen Monomer durch z. B. Polykondensation einer ω-Hydroxycarbonsäure 1 oder durch Ringöffnungspolymerisation cyclischer Ester (Lactone) 2 hergestellt.

Verzweigte und vernetzte Polyester werden bei der Polykondensation von drei- oder mehrwertigen Alkoholen mit polyfunktionellen Carbonsäuren erhalten. Zu den Polyestern werden allgemein auch die Polycarbonate (Polyester der Kohlensäure) gerechnet. AB-Typ-Polyester (I) sind u. a. Polyglykolsäuren, Polymilchsäuren, Polyhydroxybuttersäure [Poly(3-hydroxybuttersäure), Poly(ε-caprolacton)e und Polyhydroxybenzoesäuren.

Rein aliphatische AA-BB-Typ-Polyester (II) sind Polykondensate aus aliphatischen Diolen und Dicarbonsäuren, die u. a. als Produkte mit endständigen Hydroxy-Gruppen (als Polydiole) für die Herstellung von Polyesterpolyurethanen eingesetzt werden [z. B. Polytetramethylenadipat]. Mengenmäßig die größte technische Bedeutung haben AA-BB-Typ-Polyester aus aliphatischen Diolen und aromatischen Dicarbonsäuren, insbesondere die Polyalkylenterephthalate, mit Polyethylenterephthalat (PET), Polybutylenterephthalat (PBT) und Poly(1,4-cyclohexandimethylenterephthalat)e (PCDT) als wichtigste Vertreter. Diese Typen von Polyestern können durch Mitverwenden anderer aromatischer Dicarbonsäuren (z. B. Isophthalsäure) bzw. durch Einsatz von Diol-Gemischen bei der Polykondensation in ihren Eigenschaften breit variiert und unterschiedlichen Anwendungsgebieten angepaßt werden.

Rein aromatische Polyester sind die Polyarylate, zu denen u. a. die Poly(4-hydroxybenzoesäure) gehören. Zusätzlich zu den bisher genannten gesättigten Polyestern lassen sich auch ungesättigte Polyester aus ungesättigten Dicarbonsäuren herstellen, die als Polyesterharze, insbesondere als ungesättigte Polyesterharze (UP-Harze), technische Bedeutung erlangt haben.

Polyester sind in der Regel Thermoplaste. Produkte auf der Basis von aromatischen Dicarbonsäuren besitzen ausgesprochenen Werkstoffcharakter. Die rein aromatischen Polyarylate zeichnen sich durch hohe Thermostabilität aus.

Als Polyurethane (PUR) werden Polymere bezeichnet, in deren Makromolekülen die Wiederholungseinheiten durch Urethan-Gruppierungen -NH-CO-O-verknüpft sind. Polyurethane werden im allgemeinen durch Polyaddition aus zwei- oder höherwertigen Alkoholen und Isocyanaten erhalten.

Je nach Wahl und stöchiometrischem Verhältnis der Ausgangsstoffe entstehen so Polyurethane mit sehr unterschiedlichen mechanischen Eigenschaften, die als Bestandteile von Klebstoffen und Lacken (Polyurethan-Harze), als lonomere, als thermoplastisches Material für Lagerteile, Rollen, Reifen, Walzen verwendet werden und als mehr oder weniger harte Elastomere in Faserform (Elastofasern, Kurzz. PUE für diese Elastan- oder Spandex-Fasern) oder als Polyether- bzw. Polyesterurethan-Kautschuk (EU bzw. AU)

Polyurethan-Schäume entstehen bei der Polyaddition, wenn Wasser und/oder Carbonsäuren zugegen sind, denn diese reagieren mit den Isocyanaten unter Abspaltung von auftreibend und Schaum-bildend wirkendem Kohlendioxid. Mit Polyalkylenglykolethern als Diolen und Wasser als Reaktionskomponente gelangt man zu Polyurethan-Weichschäumen, mit Polyolen und Treibgasen aus FCKW (bes. R 11) erhält man Polyurethan-Hartschaumstoffe und Struktur- oder Integralschaumstoffe. Zusätzlich benötigte Hilfsstoffe sind hier z. B. Katalysatoren, Emulgatoren, Schaumstabilisatoren (bes. Polysiloxan-Polyether-Copolymere), Pigmente, Alterungs- und Flammschutzmittel. Zur Herst. von auch kompliziert geformten Gegenständen aus Polyurethan-Schaum hat man in den 70er Jahren die sogenannte RIM-Technik entwickelt (reaction injection molding = Reaktionsspritzguß). Das RIM-Verf. beruht auf raschem Dosieren und Mischen der Komponenten, Injektion des reaktiven Gemisches in die Form und schnellem Aushärten; die Cycluszeit beträgt nur wenige Minuten. Mittels RIM-Technik werden u. a. Autokarosserieteile, Schuhsohlen, Fensterprofile und Fernsehgehäuse erzeugt.

Polyvinylalkohole (PVAL, gelegentlich auch PVOH) ist dabei die Bezeichnung für Polymere der allgemeinen Struktur 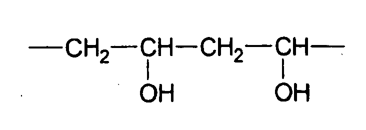 die in geringen Anteilen (ca. 2%) auch Struktureinheiten des Typs 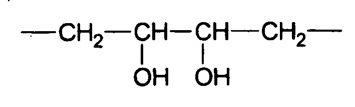 enthalten.

Handelsübliche Polyvinylalkohole werden als weiß-gelbliche Pulver oder Granulate mit Polymerisationsgraden im Bereich von ca. 100 bis 2500 (Molmassen von ca. 4000 bis 100.000 g/mol) angeboten. Charakterisiert werden die Polyvinylalkohole von Seiten der Hersteller durch Angabe des Polymerisationsgrades des Ausgangspolymeren, des Hydrolysegrades, der Verseifungszahl bzw. der Lösungsviskosität.

Polyvinylalkohole sind abhängig vom Hydrolysegrad löslich in Wasser und wenigen stark polaren organischen Lösungsmitteln (Formamid, Dimethylformamid, Dimethylsulfoxid); von (chlorierten) Kohlenwasserstoffen, Estern, Fetten und Ölen werden sie nicht angegriffen. Polyvinylalkohole werden als toxikologisch unbedenklich eingestuft und sind biologisch zumindest teilweise abbaubar. Die Wasserlöslichkeit kann man durch Nachbehandlung mit Aldehyden (Acetalisierung), durch Komplexierung mit Ni- oder Cu-Salzen oder durch Behandlung mit Dichromaten, Borsäure oder Borax verringern. Die Beschichtungen aus Polyvinylalkohol sind weitgehend undurchdringlich für Gase wie Sauerstoff, Stickstoff, Helium, Wasserstoff, Kohlendioxid, lassen jedoch Wasserdampf hindurchtreten.

Vorzugsweise werden als Materialien für die Behälter Polyvinylalkohole eines bestimmten Molekulargewichtsbereichs eingesetzt, wobei erfindungsgemäß bevorzugt ist, daß der wasserlösliche oder wasserdispergierbare Behälter einen Polyvinylalkohol umfaßt, dessen Molekulargewicht im Bereich von 10.000 bis 100.000 gmol⁻¹, vorzugsweise von 11.000 bis 90.000 gmol⁻¹, besonders bevorzugt von 12.000 bis 80.000 gmol⁻¹ und insbesondere von 13.000 bis 70.000 gmol⁻¹ liegt.

In einer besonders bevorzugten Ausführungsform der vorliegenden Erfindung besteht das polymere Trägermaterial der Partikel wenigstens anteilsweise aus Ethylen/Vinylacetat-Copolymer. Ein weiterer bevorzugter Gegenstand der vorliegenden Anmeldung ist daher ein Duftabgabesystem, **dadurch gekennzeichnet, daß** das polymere Trägermaterial mindestens 10 Gew.-%, vorzugsweise mindestens 30 Gew.-%, besonders bevorzugt mindestens 70 Gew.-% EthylenNinylacetat-Copolymer enthält, vorzugsweise vollständig aus Ethylen/Vinylacetat-Copolymer hergestellt ist.

Ethylen/Vinylacetat-Copolymere ist die Bezeichnung für Copoylmere aus Ethylen und Vinylacetat. Die Herstellung dieses Polymers erfolgt grundsätzlich in einem der Herstellung von Polyethylen mit niedriger Dichte (LDPE; low density polyethylene) vergleichbaren Verfahren. Mit einem zunehmenden Anteil an Vinylacetat wird die Kristallinität des Polyethylens unterbrochen und auf diese Weise die Schmelz- und Erweichungspunkte bzw. die Härte der resultierenden Produkte herabgesetzt. Das Vinylacetat macht das Copolymer zudem polarer und verbessert damit dessen Adhäsion an polare Substrate.

Die vorstehend beschriebenen EthylenNinylacetat-Copolymere sind kommerziell breit verfügbar, beispielsweise unter dem Warenzeichen Elvax^{®} (Dupont). Im Rahmen der vorliegenden Erfindung besonders geeignete Polyvinylalkohole sind beispielsweise Elvax^{®} 265, Elvax^{®} 240, Elvax^{®} 205 W, Elvax^{®} 200 W sowie Elvax^{®} 360.

Einige besonders geeignete Copolymere und deren physikalische Eigenschaften sind der nachstehenden Tabelle zu entnehmen:

| Produktname | Gew.-% Vinylacetat (bezogen auf das Gesamtgewicht) | Schmelzpunkt |
|---|---|---|
| Elvax^{®} 40W | 40 | 47°C |
| Elvax^{®} 150 | 33 | 63°C |
| Elvax^{®} 265 | 28 | 75°C |
| Elvax^{®} 240 | 28 | 74°C |
| Elvax^{®} 205 W | 28 | 72°C |
| **Elvax^{®} 200 W** | 28 | 71°C |
| Elvax^{®} 360 | 25 | 78°C |
| Elvax^{®} 460 | 18 | 88°C |
| Elvax^{®} 660 | 12 | 96°C |
| Elvax^{®} 760 | 9 | 100°C |

Im Rahmen der vorliegenden Erfindung, insbesondere im Bereich der Beduftung der Innenräume sind Duftabgabesysteme besonders bevorzugt, in welchen als polymeres Trägermaterial EthylenNinylacetat-Copolymer eingesetzt wird und dieses Copolymer 5 bis 50 Gew.-% Vinylacetat, vorzugsweise 10 bis 40 Gew.-% Vinylacetat und insbesondere 20 bis 30 Gew.-% Vinylacetat, jeweils bezogen auf das Gesamtgewicht des Copolymers, enthält.

Erfindungsgemäße Duftabgabesysteme enthalten die polymeren Trägermaterialien in Form von Partikeln. Die Raumform dieser Partikel wird lediglich durch die technischen Möglichkeiten bei deren Herstellung beschränkt. Als Raumform kommen damit alle sinnvoll handhabbaren Ausgestaltungen in Betracht, beispielsweise also Würfel, Quader und entsprechende Raumelemente mit ebenen Seitenflächen sowie insbesondere zylinderförmige Ausgestaltungen mit kreisförmigem oder ovalem Querschnitt. Diese letzte Ausgestaltung umfaßt dabei tablettenförmige Teilchen bis hin zu kompakten Zylinderstücken mit einem Verhältnis von Höhe zu Durchmesser oberhalb 1. Weitere mögliche Raumformen sind Kugeln, Halbkugeln oder "gestreckte Kugeln" in Form ellipsoider Kapseln ebenso wie reguläre Polyeder, beispielsweise Tetraeder, Hexaeder, Oktaeder, Dodekaeder, Ikosaeder. Denkbar sind weiterhin sternförmige Ausbildungen mit drei, vier, fünf, sechs oder mehr Spitzen oder völlig unregelmäßige Körper, die beispielsweise motivisch ausgestaltet sein können. Als Motive eignen sich in Abhängigkeit von dem Einsatzgebiet der erfindungsgemäßen Mittel zum Beispiel Tierfiguren, wie Hunde, Pferde oder Vögel, florale Motive oder die Darstellung von Früchten. Die motivische Ausgestaltung kann sich aber auch auf unbelebte Gegenstände wie Fahrzeuge, Werkzeuge, Haushaltsgegenstände oder Bekleidung beziehen. Die Oberfläche der Feststoffteilchen kann in Abhängigkeit von der Art des gewählten Herstellungsverfahrens und/oder einer gewählten Beschichtung Unebenheiten aufweisen. Aufgrund der zahlreichen Ausgestaltungsmöglichkeiten für die Partikel zeichnen sich die erfindungsgemäßen Mittel nicht nur durch Vorteile bei ihrer Herstellung aus. Aufgrund der vielfältigen Ausgestaltungsformen sind die duftstoffhaltigen Partikel zudem für den Verbraucher optisch deutlich wahrnehmbar und ermöglichen durch die gezielte räumliche Gestaltung dieser Partikel ein für die Produktakzeptanz besonders vorteilhafte Visualisierung der in den erfindungsgemäßen Mittel enthaltenen Duftstoffe, bzw. weiterer optional in diesen Mitteln enthaltenen Aktivsubstanzen. So kann durch die optisch wahrnehmbare Mehrphasigkeit dieser Mittel beispielsweise die unterschiedliche Wirkweise einzelner Aktivsubstanzen (z.B. Reinigungs- und Zusatzfunktionen wie Glasschutz, Silberschutz etc.) verdeutlicht werden.

Als Partikel werden im Rahmen der vorliegenden Anmeldung Teilchen zusammengefaßt, welche eine bei Raumtemperatur feste, das heißt formstabile, nicht fließfähige Konsistenz aufweisen. Bevorzugte Partikel weisen einen mittleren Durchmesser von 0,5 bis 20 mm, vorzugsweise von 1 bis 10 mm und insbesondere von 3 bis 6 mm auf.

Die Konfektionierung der polymeren Trägermaterialien zu den zuvor beschriebenen Partikeln kann auf alle dem Fachmann zur Verarbeitung dieser Substanzen bekannten Verfahren erfolgen. Bevorzugt werden im Rahmen der vorliegenden Erfindung die Extrusion das Spritzgießen und das Versprühen zu Polymergranulaten bevorzugt.

### Duftstoffe

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die allgemeine Beschreibung der einsetzbaren Parfüme (siehe oben) stellt dabei allgemein die unterschiedlichen Substanzklassen von Riechstoffen dar. Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Durch eine geeignete Auswahl der genannten Duftstoffe bzw. Parfümöle kann auf diese Weise für erfindungsgemäße Mittel sowohl der Produktgeruch unmittelbar beim Öffnen des fabrikneuen Mittels als auch der Gebrauchsduft, beispielsweise beim Einsatz in einer Geschirrspülmaschine, beeinflußt werden. Diese Dufteindrücke können selbstverständlich gleich sein, können sich aber auch unterscheiden. Für den letzteren Geruchseindruck ist die Verwendung haftfesterer Riechstoffe vorteilhaft, während zur Produktbeduftung auch leichterflüchtige Riechstoffe einsetzbar sind. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Bevorzugt werden die Kunststoffpartikel bei einer Temperatur von 15 bis 30°C, vorzugsweise von 20 bis 25°C, mit dem ausgewählten Duftstoff beladen. Hierzu werden die Partikel mit der entsprechenden Menge des Duftstoffs versetzt und durchmischt. In jedem Fall sollte die Temperatur aber unterhalb der Schmelz- oder Zersetzungstemperatur des Kunststoffs und auch unterhalb des Flammpunkts des Parfumöls liegen. Der Duftstoff wird vorrangig durch Adhäsions-, Diffusions- und/oder Kapillarkräfte vom polymeren Trägermaterial oder von im Partikel enthaltenen weiteren Parfümträgermaterialien aufgenommen, wobei diese im Laufe dieses Vorgangs geringfügig quellen können.

### Weitere Aktivsubstanzen

Wie zuvor erwähnt, können erfindungsgemäße Mittel außer den zur Beduftung und Desodorierung notwendigen Bestandteilen weitere Aktivsubstanzen enthalten. Von den Mitteln, welche ausschließlich der Beduftung dienen, lassen sich demnach weitere Produktgruppen unterscheiden, welche zusätzlich zu den vorgenannten erfindungsgemäßen Bestandteilen weitere bevorzugte Substanzen enthalten.

### Farbstoffe

Eine erste dieser optional einsetzbaren bevorzugten Substanzen sind die Farbstoffe. Hierzu eignen sich generell sämtliche Farbstoffe, die dem Fachmann als geeignet zum Einfärben von Kunststoffen bzw. als löslich in Parfumölen bekannt sind. Es ist bevorzugt, den Farbstoff entsprechend des verwendeten Duftstoffs auszuwählen; beispielsweise weisen Partikel mit Zitronenduft vorzugsweise eine gelbe Farbe auf, während für Partikel mit Apfel- oder Kräuterduft eine grüne Farbe bevorzugt wird. Bevorzugte Farbstoffe besitzen eine hohe Lagerstabilität und Unempfindlichkeit gegenüber den übrigen Inhaltsstoffen der Mittel und gegen Licht. Werden die erfindungsgemäßen Mittel im Zusammenhang mit der Textil- oder Geschirreinigung eingesetzt, sollten die eingesetzten Farbstoffe keine ausgeprägte Substantivität gegenüber Textilfaser, Glas, Kunststoffgeschirr oder Keramik aufweisen, um diese nicht anzufärben.

Geeignete Farbstoffe und Farbstoffgemische sind unter verschiedenen Handelsnamen kommerziell erhältlich und werden unter anderem von den Firmen BASF AG, Ludwigshafen, Bayer AG, Leverkusen, Clariant GmbH, DyStar Textilfarben GmbH & Co. Deutschland KG, Les Colorants Wackherr SA und Ciba Specialty Chemicals angeboten. Zu den geeigneten fettlöslichen Farbstoffen und Farbstoffgemischen zählen beispielsweise Solvent Blue 35, Solvent Green 7, Solvent Orange 1 (Orange au Gras-W-2201), Sandoplast Blau 2B, Fettgelb 3G, Iragon® Red SRE 122, Iragon® Green SGR 3, Solvent Yellow 33 und Solvent Yellow 16, es können aber auch andere Farbstoffe enthalten sein.

In einer bevorzugten Ausführungsform besitzt der Farbstoff neben seiner ästhetischen Wirkung zusätzlich eine Indikatorfunktion. Hierdurch wird dem Konsumenten der aktuelle Verbrauchszustand des Deodorants angezeigt, so daß er neben dem fehlenden Dufteindruck, der beispielsweise auch auf einem Gewöhnungseffekt seitens des Benutzers beruhen kann, ein weiteres zuverlässiges Anzeichen erhält, wann das Deodorant durch ein neues zu ersetzen ist.

Die Indikatorwirkung kann auf verschiedenen Wegen erzielt werden: Einerseits kann ein Farbstoff verwendet werden, der im Laufe der Anwendungsdauer aus den Partikeln entweicht. Dies kann zum Beispiel durch die im Geschirrspülmittel enthaltenen Inhaltsstoffe bewirkt werden. Hierzu muß ein Farbstoff eingesetzt werden, der gut an den Partikeln haftet bzw. nur langsam aus ihnen herausdiffundiert, um zu gewährleisten, daß die Entfärbung nicht zu früh, nämlich wenn der Duftstoff noch nicht verbraucht ist, beendet ist. Andererseits kann aber auch durch eine chemische Reaktion oder thermische Zersetzung ein Farbumschlag hervorgerufen werden.

### Antimikrobielle Wirkstoffe, Germizide, Fungizide

Weitere bevorzugte Bestandteile erfindungsgemäßer Mittel sind Substanzen wie antimikrobielle Wirkstoffe, Germizide, Fungizide, Antioxidantien oder Korrosionsinhibitoren, mit deren Hilfe sich Zusatznutzen, wie beispielsweise die Desinfektion oder der Korrosionsschutz realisieren lassen.

Zur Bekämpfung von Mikroorganismen können die erfindungsgemäßen Mittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw.. Wichtige Stoffe aus diesen Gruppen sind beispielsweise Benzalkoniumchloride, Alkylarlylsulfonate, Halogenphenole und Phenolmercuriacetat.

### Antioxidantien

Um unerwünschte, durch Sauerstoffeinwirkung und andere oxidative Prozesse verursachte Veränderungen an den erfindungsgemäßen Mitteln oder den beispielsweise behandelten Textilien zu verhindern, können die Mittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite und Phosphonate.

Werden die erfindungsgemäßen Mittel in Geschirrspülmaschinen zum Einsatz gebracht, so können diese Mittel zum Schutze des Spülgutes oder der Maschine Korrosionsinhibitoren enthalten, wobei besonders Silberschutzmittel im Bereich des maschinellen Geschirrspülens eine besondere Bedeutung haben. Einsetzbar sind die bekannten Substanzen des Standes der Technik. Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders sauerstoff- und stickstoffhaltige organische redoxaktive Verbindungen, wie zwei- und dreiwertige Phenole, z. B. Hydrochinon, Brenzkatechin, Hydroxyhydrochinon, Gallussäure, Phloroglucin, Pyrogallol bzw. Derivate dieser Verbindungsklassen, eingesetzt. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder - komplexe, besonders bevorzugt der Cobalt(ammin)-Komplexe, der Cobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

Anstelle von oder zusätzlich zu den vorstehend beschriebenen Silberschutzmitteln, beispielsweise den Benzotriazolen, können in den erfindungsgemäßen Mittel redoxaktive Substanzen eingesetzt werden. Diese Substanzen sind vorzugsweise anorganische redoxaktive Substanzen aus der Gruppe der Mangan-, Titan-, Zirkonium-, Hafnium-, Vanadium-, Cobalt- und Cer-Salze und/oder -Komplexe, wobei die Metalle vorzugsweise in einer der Oxidationsstufen II, III, IV, V oder VI vorliegen.

Die verwendeten Metallsalze bzw. Metallkomplexe sollen zumindest teilweise in Wasser löslich sein. Die zur Salzbildung geeigneten Gegenionen umfassen alle üblichen ein-, zwei-, oder dreifach negativ geladenen anorganischen Anionen, z. B. Oxid, Sulfat, Nitrat, Fluorid, aber auch organische Anionen wie z. B. Stearat.

Metallkomplexe im Sinne der Erfindung sind Verbindungen, die aus einem Zentralatom und einem oder mehreren Liganden sowie gegebenenfalls zusätzlich einem oder mehreren der o.g. Anionen bestehen. Das Zentralatom ist eines der o.g. Metalle in einer der o.g. Oxidationsstufen. Die Liganden sind neutrale Moleküle oder Anionen, die ein- oder mehrzähnig sind; der Begriff "Liganden" im Sinne der Erfindung ist z.B. in "Römpp Chemie Lexikon, Georg Thieme Verlag Stuttgart/New York, 9. Auflage, 1990, Seite 2507" näher erläutert. Ergänzen sich in einem Metallkomplex die Ladung des Zentralatoms und die Ladung des/der Liganden nicht auf Null, so sorgt, je nachdem, ob ein kationischer oder ein anionischer Ladungsüberschuß vorliegt, entweder eines oder mehrere der o.g. Anionen oder ein oder mehrere Kationen, z. B. Natrium-, Kalium-, Ammoniumionen, für den Ladungsausgleich. Geeignete Komplexbildner sind z.B. Citrat, Acetylacetonat oder 1-Hydroxyethan-1,1-diphosphonat.

Die in der Chemie geläufige Definition für "Oxidationsstufe" ist z.B. in "Römpp Chemie Lexikon, Georg Thieme Verlag Stuttgart/New York, 9. Auflage, 1991, Seite 3168" wiedergegeben.

Besonders bevorzugte Metallsalze und/oder Metallkomplexe sind ausgewählt aus der Gruppe MnSO₄, Mn(II)-citrat, Mn(II)-stearat, Mn(II)-acetylacetonat, Mn(II)-[1-Hydroxyethan-1,1-diphosphonat], V₂O₅, V₂O₄, VO₂, TiOSO₄, K₂TiF₆, K₂ZrF₆, CoSO₄, Co(NO₃)₂, Ce(NO₃)₃ sowie deren Gemische, so daß bevorzugte erfindungsgemäße Mittel dadurch gekennzeichnet sind, daß die Metallsalze und/oder Metallkomplexe ausgewählt sind aus der Gruppe MnSO₄, Mn(II)-citrat, Mn(II)-stearat, Mn(II)-acetylacetonat, Mn(II)-[1-Hydroxyethan-1,1-diphosphonat], V₂O₅, V₂O₄, VO₂, TiOSO₄, K₂TiF₆, K₂ZrF₆, CoSO₄, Co(NO₃)₂, Ce(NO₃)₃,

Bei diesen Metallsalzen bzw. Metallkomplexen handelt es sich im allgemeinen um handelsübliche Substanzen, die zum Zwecke des Silberkorrosions-Schutzes ohne vorherige Reinigung in den erfindungsgemäßen Mitteln eingesetzt werden können. So ist z.B. das aus der SO₃-Herstellung (Kontaktverfahren) bekannte Gemisch aus fünf- und vierwertigem Vanadium (V₂O₅, VO₂, V₂O₄) geeignet, ebenso wie das durch Verdünnen einer Ti(SO₄)₂-Lösung entstehende Titanylsulfat, TiOSO₄.

Die genannten Metallsalze und/oder Metallkomplexe sind in den erfindungsgemäßen Mitteln, vorzugsweise in einer Menge von 0,05 bis 6 Gew.-%, vorzugsweise 0,2 bis 2,5 Gew.-%, bezogen auf das gesamte Mittel ohne den Behälter, enthalten

### Mittel zur Verhinderung von Glaskorrosion

Ein wichtiges Kriterium zur Beurteilung eines maschinellen Geschirrspülmittels ist neben dessen Reinigungsleistung das optische Erscheinungsbild des trockenen Geschirrs nach erfolgter Reinigung. Eventuell auftretende Calciumcarbonat-Ablagerungen auf Geschirr oder im Maschineninnenraum können beispielsweise die Kundenzufriedenheit beeinträchtigen und haben damit ursächlichen Einfluß auf den wirtschaftlichen Erfolg eines derartigen Reinigungsmittels. Ein weiteres seit langem bestehendes Problem beim maschinellen Geschirrspülen ist die Korrosion von Glasspülgut, die sich in der Regel durch Auftreten von Trübungen, Schlieren und Kratzern, aber auch durch ein Irisieren der Glasoberfläche äußern kann. Die beobachteten Effekte beruhen dabei im wesentlichen auf zwei Vorgängen, zum einen dem Austritt von Alkali- und Erdalkaliionen aus dem Glas in Verbindung mit einer Hydrolyse des Silikat-Netzwerks, zum anderen in einer Ablagerung silikatischer Verbindungen auf der Glasoberfläche.

Die genannten Probleme können mit den erfindungsgemäßen Mitteln gelöst werden, wenn zusätzlich zu den vorstehend genannten zwingenden und gegebenenfalls optionalen Inhaltsstoffen bestimmte Glaskorrosionsinhibitoren in die Mittel inkorporiert werden. Bevorzugte erfindungsgemäße Mittel enthalten daher zusätzlich ein oder mehrere Magnesium- und/oder Zinksalze und/oder Magnesium- und/oder Zinkkomplexe.

Eine bevorzugte Klasse von Verbindungen, die zur Verhinderung der Glaskorrosion den erfindungsgemäßen Mitteln zugesetzt werden können, sind unlösliche Zinksalze. Diese können sich während des Geschirrspülvorgangs an der Glasoberfläche anlagern und verhindern dort das in Lösung gehen von Metallionen aus dem Glasnetzwerk sowie die Hydrolyse der Silikate. Zusätzlich verhindern diese unlöslichen Zinksalze auch die Ablagerung von Silikat auf der Glasoberfläche, so daß das Glas vor den vorstehend geschilderten Folgen geschützt ist.

Unlösliche Zinksalze im Sinne dieser bevorzugten Ausführungsform sind Zinksalze, die eine Löslichkeit von maximal 10 Gramm Zinksalz pro Liter Wasser bei 20°C besitzen. Beispiele für erfindungsgemäß besonders bevorzugte unlösliche Zinksalze sind Zinksilikat, Zinkcarbonat, Zinkoxid, basisches Zinkcarbonat (Zn₂(OH)₂CO₃), Zinkhydroxid, Zinkoxalat, Zinkmonophosphat (Zn₃(PO₄)₂), und Zinkpyrophosphat (Zn₂(P₂O₇)).

Die genannten Zinkverbindungen werden in den erfindungsgemäßen Mitteln in Mengen eingesetzt, die einen Gehalt der Mittel an Zinkionen zwischen 0,02 und 10 Gew.-%, vorzugsweise zwischen 0,1 und 5,0 Gew.-% und insbesondere zwischen 0,2 und 1,0 Gew.-%, jeweils bezogen auf das Mittel ohne den Behälter, bewirken. Der exakte Gehalt der Mittel am Zinksalz bzw. den Zinksalzen ist naturgemäß abhängig von der Art der Zinksalze - je weniger löslich das eingesetzte Zinksalz ist, umso höher sollte dessen Konzentration in den erfindungsgemäßen Mitteln sein.

Eine weitere bevorzugte Klasse von Verbindungen sind Magnesium- und/oder Zinksalz(e) mindestens einer monomeren und/oder polymeren organischen Säure. Diese bewirken, daß auch bei wiederholter Benutzung die Oberflächen gläsernen Spülguts nicht korrosiv verändert, insbesondere keine Trübungen, Schlieren oder Kratzer aber auch kein Irisieren der Glasoberflächen verursacht werden.

Obwohl erfindungsgemäß alle Magnesium- und/oder Zinksalz(e) monomerer und/oder polymerer organischer Säuren in den beanspruchten Mitteln enthalten sein können, werden doch, wie vorstehend beschrieben, die Magnesium- und/oder Zinksalze monomerer und/oder polymerer organischer Säuren aus den Gruppen der unverzweigten gesättigten oder ungesättigten Monocarbonsäuren, der verzweigten gesättigten oder ungesättigten Monocarbonsäuren, der gesättigten und ungesättigten Dicarbonsäuren, der aromatischen Mono-, Di- und Tricarbonsäuren, der Zuckersäuren, der Hydroxysäuren, der Oxosäuren, der Aminosäuren und/oder der polymeren Carbonsäuren bevorzugt. Innerhalb dieser Gruppen werden im Rahmen der vorliegenden Erfindung wiederum die in der Folge genannten Säuren bevorzugt:

Das Spektrum der erfindungsgemäß bevorzugten Zinksalze organischer Säuren, vorzugsweise organischer Carbonsäuren, reicht von Salzen, die in Wasser schwer oder nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinkoleat und das Zinkstearat, zu der Gruppe der löslichen Zinksalze gehören beispielsweise das Zinkformiat, das Zinkacetat, das Zinklactat und das Zinkgluconat:

In einer weiteren bevorzugten Ausführungsform der vorliegenden Erfindung enthalten die erfindungsgemäßen Mittel wenigstens ein Zinksalz, jedoch kein Magnesiumsalz einer organischen Säure, wobei es sich vorzugsweise um mindestens ein Zinksalz einer organischen Carbonsäure, besonders bevorzugt um ein Zinksalz aus der Gruppe Zinkstearat, Zinkoleat, Zinkgluconat, Zinkacetat, Zinklactat und/oder Zinkcitrat handelt. Auch Zinkricinoleat, Zinkabietat und Zinkoxalat sind bevorzugt.

Ein im Rahmen der vorliegenden Erfindung bevorzugtes Mittel enthält Zinksalz in Mengen von 0,1 bis 5 Gew.-%, vorzugsweise von 0,2 bis 4 Gew.-% und insbesondere von 0,4 bis 3 Gew.-%, bzw. Zink in oxidierter Form (berechnet als Zn²⁺) in Mengen von 0,01 bis 1 Gew.-%, vorzugsweise von 0,02 bis 0,5 Gew.-% und insbesondere von 0,04 bis 0,2 Gew.-%, jeweils bezogen auf das Mittel ohne den Behälter.

Ein weiterer bevorzugter Gegenstand der vorliegenden Anmeldung ist daher ein Duftabgabesystem, welches weitere Aktivsubstanzen, insbesondere Aktivsubstanzen aus der Gruppe der Parfümträger, Farbstoffe, antimikrobiellen Wirkstoffe, Germizide, Fungizide, Antioxidantien oder Korrosionsinhibitoren enthalten.

### Bleichmittel

Zusätzlich zu den vorgenannten Aktivsubstanzen können die erfindungsgemäßen Mittel, insbesondere Mittel für den Einsatz in Geschirrspülmaschinen, Textilwaschmaschinen oder -trockern, selbstverständlich alle üblicherweise in Mitteln für die Textil- oder Geschirreinigung bzw. die Textil- oder Geschirrpflege enthaltenen Aktivsubstanzen aufweisen, wobei Substanzen aus der Gruppe der Bleichmittel, Bleichaktivatoren, Polymere, Gerüststoffe, Tenside, Enzyme, Elektrolyte, pH-Stellmittel, Duftstoffe, Parfümträger, Farbstoffe, Hydrotrope, Schauminhibitoren, Antiredepositionsmittel, optischen Aufheller, Vergrauungsinhibitoren, Einlaufverhinderer, Knitterschutzmittel, Farbübertragungs-inhibitoren, antimikrobiellen Wirkstoffe, Germizide, Fungizide, Antioxidantien, Korrosionsinhibitoren, Antistatika, Phobier- und Imprägniermittel, Quell- und Schiebefestmittel, nichtwässrigen Lösungsmittel, Weichspüler, Proteinhydrolysate, sowie UV-Absorber besonders bevorzugt sind. Derartige Kombinationsprodukte eignen sich dann neben der wiederholten Beduftung auch zur ein- oder mehrmaligen Pflege oder Reinigung von Textilien oder Geschirr.

Als wichtige Bestandteile von Wasch- oder Reinigungsmitteln können in den erfindungsgemäßen Mitteln neben anderen Bestandteilen Bleichmittel und Bleichaktivatoren enthalten sein. Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Reinigungsmittelformkörper für das maschinelle Geschirrspülen können auch Bleichmittel aus der Gruppe der organischen Bleichmittel enthalten. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamidoperoxycapronsäure, N-nonenylamidoperadipinsäure und N-nonenylamidopersuccinate, und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Werden die erfindungsgemäßen Mittel in Kombination mit maschinellen Geschirrspülmitteln eingesetzt, so können diese Bleichaktivatoren enthalten, um beim Reinigen bei Temperaturen von 60 °C und darunter eine verbesserte Bleichwirkung zu erreichen. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Weitere im Rahmen der vorliegenden Anmeldung bevorzugt eingesetzte Bleichaktivatoren sind Verbindungen aus der Gruppe der kationischen Nitrile, insbesondere kationische Nitrile der Formel 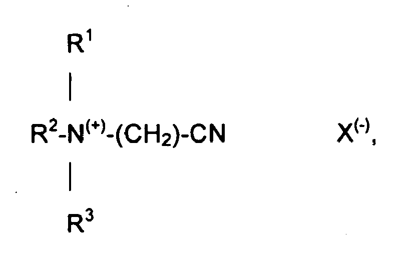 in der R¹ für -H, -CH₃, einen C₂₋₂₄-Alkyl- oder -Alkenylrest, einen substituierten C₂₋₂₄-Alkyl- oder -Alkenylrest mit mindestens einem Substituenten aus der Gruppe -Cl, -Br, -OH, -NH₂, -CN, einen Alkyl- oder Alkenylarylrest mit einer C₁₋₂₄-Alkylgruppe, oder für einen substituierten Alkyl- oder Alkenylarylrest mit einer C₁₋₂₄-Alkylgruppe und mindestens einem weiteren Substituenten am aromatischen Ring steht, R² und R³ unabhängig voneinander ausgewählt sind aus -CH₂-CN, -CH₃, -CH₂-CH₃, -CH₂-CH₂-CH₃, -CH(CH₃)-CH₃, -CH₂-OH, -CH₂-CH₂-OH, - CH(OH)-CH₃, -CH₂-CH₂-CH₂-OH, -CH₂-CH(OH)-CH₃, -CH(OH)-CH₂-CH₃, - (CH₂CH₂-O)ₙH mit n = 1, 2, 3, 4, 5 oder 6 und X ein Anion ist.

In besonders bevorzugten erfindungsgemäßen Mitteln ist ein kationisches Nitril der Formel 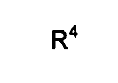 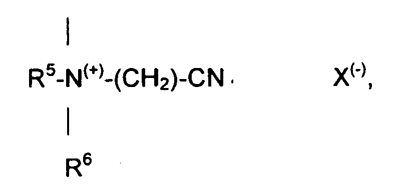 enthalten, in der R⁴, R⁵ und R⁶ unabhängig voneinander ausgewählt sind aus - CH₃, -CH₂-CH₃, -CH₂-CH₂-CH₃, -CH(CH₃)-CH₃, wobei R⁴ zusätzlich auch -H sein kann und X ein Anion ist, wobei vorzugsweise R⁵ = R⁶ = -CH₃ und insbesondere R⁴ = R⁵ = R⁶ = -CH₃ gilt und Verbindungen der Formeln (CH₃)₃N⁽⁺⁾CH₂-CN X⁻, (CH₃CH₂)₃N⁽⁺⁾CH₂-CN X⁻, (CH₃CH₂CH₂)₃N⁽⁺⁾CH₂-CN X⁻, (CH₃CH(CH₃))₃N⁽⁺⁾CH₂-CN X⁻, oder (HO-CH₂-CH₂)₃N⁽⁺⁾CH₂-CN X⁻ besonders bevorzugt sind, wobei aus der Gruppe dieser Substanzen wiederum das kationische Nitril der Formel (CH₃)₃N⁽⁺⁾CH₂-CN X⁻, in welcher X⁻ für ein Anion steht, das aus der Gruppe Chlorid, Bromid, Iodid, Hydrogensulfat, Methosulfat, p-Toluolsulfonat (Tosylat) oder Xylolsulfonat ausgewählt ist, besonders bevorzugt wird.

Zusätzlich zu den konventionellen Bleichaktivatoren oder an deren Stelle können auch sogenannte Bleichkatalysatoren in die Mittel eingearbeitet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

### Tenside

Bevorzugte Mittel enthalten im Rahmen der vorliegenden Anmeldung ein oder mehrere Tensid(e) aus den Gruppen der anionischen, nichtionischen, kationischen und/oder amphoteren Tenside.

Als Aniontenside in Säureform werden bevorzugt ein oder mehrere Stoffe aus der Gruppe der Carbonsäuren, der Schwefelsäurehalbester und der Sulfonsäuren, vorzugsweise aus der Gruppe der Fettsäuren, der Fettalkylschwefelsäuren und der Alkylarylsulfonsäuren, eingesetzt. Um ausreichende oberflächenaktive Eigenschaften aufzuweisen, sollten die genannten Verbindungen dabei über längerkettige Kohlenwasserstoffreste verfügen, also im Alkyl- oder Alkenylrest mindestens 6 C-Atome aufweisen. Üblicherweise liegen die C-Kettenverteilungen der Aniontenside im Bereich von 6 bis 40, vorzugsweise 8 bis 30 und insbesondere 12 bis 22 Kohlenstoffatome.

Carbonsäuren, die in Form ihrer Alkalimetallsalze als Seifen in Wasch- und Reinigungsmitteln Verwendung finden, werden technisch größtenteils aus nativen Fetten und Ölen durch Hydrolyse gewonnen. Während die bereits im vergangenen Jahrhundert durchgeführte alkalische Verseifung direkt zu den Alkalisalzen (Seifen) führte, wird heute großtechnisch zur Spaltung nur Wasser eingesetzt, das die Fette in Glycerin und die freien Fettsäuren spaltet. Großtechnisch angewendete Verfahren sind beispielsweise die Spaltung im Autoklaven oder die kontinuierliche Hochdruckspaltung. Im Rahmen der vorliegenden Erfindung als Aniontensid in Säureform einsetzbare Carbonsäuren sind beispielsweise Hexansäure (Capronsäure), Heptansäure (Önanthsäure), Octansäure (Caprylsäure), Nonansäure (Pelargonsäure), Decansäure (Caprinsäure), Undecansäure usw.. Bevorzugt ist im Rahmen der vorliegenden Verbindung der Einsatz von Fettsäuren wie Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure), Tetracosansäure (Lignocerinsäure), Hexacosansäure (Cerotinsäure), Triacotansäure (Melissinsäure) sowie der ungesättigten Spezies 9c-Hexadecensäure (Palmitoleinsäure), 6c-Octadecensäure (Petroselinsäure), 6t-Octadecensäure (Petroselaidinsäure), 9c-Octadecensäure (Ölsäure), 9t-Octadecensäure ((Elaidinsäure), 9c,12c-Octadecadiensäure (Linolsäure), 9t,12t-Octadecadiensäure (Linolaidinsäure) und 9c,12c,15c-Octadecatriensäure (Linolensäure). Aus Kostengründen ist es bevorzugt, nicht die reinen Spezies einzusetzen, sondern technische Gemische der einzelnen Säuren, wie sie aus der Fettspaltung zugänglich sind. Solche Gemische sind beispielsweise Kokosölfettsäure (ca. 6 Gew.-% C₈, 6 Gew.-% C₁₀, 48 Gew.-% C₁₂, 18 Gew.-% C₁₄, 10 Gew.-% C₁₆, 2 Gew.-% C₁₈, 8 Gew.-% C_{18'}, 1 Gew.-% C_{18"}), Palmkernölfettsäure (ca. 4 Gew.-% C₈, 5 Gew.-% C₁₀, 50 Gew.-% C₁₂, 15 Gew.-% C₁₄, 7 Gew.-% C₁₆, 2 Gew.-% C₁₈, 15 Gew.-% C_{18'}, 1 Gew.-% C_{18"}), Talgfettsäure (ca. 3 Gew.-% C₁₄, 26 Gew.-% C₁₆, 2 Gew.-% C_{16'}, 2 Gew.-% C₁₇, 17 Gew.-% C₁₈, 44 Gew.-% C_{18'}, 3 Gew.-% C_{18"}, 1 Gew.-% C_{18"'}), gehärtete Talgfettsäure (ca. 2 Gew.-% C₁₄, 28 Gew.-% C₁₆, 2 Gew.-% C₁₇, 63 Gew.-% C₁₈, 1 Gew.-% C_{18'}), technische Ölsäure (ca. 1 Gew.-% C₁₂, 3 Gew.-% C₁₄, 5 Gew.-% C₁₆, 6 Gew.-% C_{16'}, 1 Gew.-% C₁₇, 2 Gew.-% C₁₈, 70 Gew.-% C_{18'}, 10 Gew.-% C_{18"}, 0,5 Gew.-% C_{18"'}), technische Palmitin/Stearinsäure (ca. 1 Gew.-% C₁₂, 2 Gew.-% C₁₄, 45 Gew.-% C₁₆, 2 Gew.-% C₁₇, 47 Gew.-% C₁₈, 1 Gew.-% C_{18'}) sowie Sojabohnenölfettsäure (ca. 2 Gew.-% C₁₄, 15 Gew.-% C₁₆, 5 Gew.-% C₁₈, 25 Gew.-% C_{18'}, 45 Gew.-% C_{18"}, 7 Gew.-% C_{18"'}).

Schwefelsäurehalbester längerkettiger Alkohole sind ebenfalls Aniontenside in ihrer Säureform und im Rahmen der vorliegenden Erfindung einsetzbar. Ihre Alkalimetall-, insbesondere Natriumsalze, die Fettalkoholsulfate, sind großtechnisch aus Fettalkoholen zugänglich, welche mit Schwefelsäure, Chlorsulfonsäure, Amidosulfonsäure oder Schwefeltrioxid zu den betreffenden Alkylschwefelsäuren umgesetzt und nachfolgend neutralisiert werden. Die Fettalkohole werden dabei aus den betreffenden Fettsäuren bzw. Fettsäuregemischen durch Hochdruckhydrierung der Fettsäuremethylester gewonnen. Der mengenmäßig bedeutendste industrielle Prozeß zur Herstellung von Fettalkylschwefelsäuren ist die Sulfierung der Alkohole mit SO₃/LuftGemischen in speziellen Kaskaden-, Fallfilm- oder Röhrenbündelreaktoren.

Eine weitere Klasse von Aniontensidsäuren, die erfindungsgemäß eingesetzt werden kann, sind die Alkyletherschwefelsäuren, deren Salze, die Alkylethersulfate, sich im Vergleich zu den Alkylsulfaten durch eine höhere Wasserlöslichkeit und geringere Empfindlichkeit gegen Wasserhärte (Löslichkeit der Ca-Salze) auszeichnen. Alkyletherschwefelsäuren werden wie die Alkylschwefelsäuren aus Fettalkoholen synthetisiert, welche mit Ethylenoxid zu den betreffenden Fettalkoholethoxylaten umgesetzt werden. Anstelle von Ethylenoxid kann auch Propylenoxid eingesetzt werden. Die nachfolgende Sulfonierung mit gasförmigem Schwefeltrioxid in Kurzzeit-Sulfierreaktoren liefert Ausbeuten über 98% an den betreffenden Alkyletherschwefelsäuren.

Auch Alkansulfonsäuren und Olefinsulfonsäuren sind im Rahmen der vorliegenden Erfindung als Aniontenside in Säureform einsetzbar. Alkansulfonsäuren können die Sulfonsäuregruppe terminal gebunden (primäre Alkansulfonsäuren) oder entlang der C-Kette enthalten (sekundäre Alkansulfonsäuren), wobei lediglich die sekundären Alkansulfonsäuren kommerzielle Bedeutung besitzen. Diese werden durch Sulfochlorierung oder Sulfoxidation linearer Kohlenwasserstoffe hergestellt. Bei der Sulfochlorierung nach Reed werden n-Paraffine mit Schwefeldioxid und Chlor unter Bestrahlung mit UV-Licht zu den entsprechenden Sulfochloriden umgesetzt, die bei Hydrolyse mit Alkalien direkt die Alkansulfonate, bei Umsetzung mit Wasser die Alkansulfonsäuren, liefern. Da bei der Sulfochlorierung Di- und Polysulfochloride sowie Chlorkohlenwasserstoffe als Nebenprodukte der radikalischen Reaktion auftreten können, wird die Reaktion üblicherweise nur bis zu Umsetzungsgraden von 30% durchgeführt und danach abgebrochen.

Ein anderer Prozeß zur Herstellung von Alkansulfonsäuren ist die Sulfoxidation, bei der n-Paraffine unter Bestrahlung mit UV-Licht mit Schwefeldioxid und Sauerstoff umgesetzt werden. Bei dieser Radikalreaktion entstehen sukzessive Alkylsulfonylradikale, die mit Sauerstoff zu den Alkylpersulfonylradikalen weiter reagieren. Die Reaktion mit unumgesetztem Paraffin liefert ein Alkylradikal und die Alkylpersulfonsäure, welche in ein Alkylperoxysulfonylradikal und ein Hydroxylradikal zerfällt. Die Reaktion der beiden Radikale mit unumgesetztem Paraffin liefert die Alkylsulfonsäuren bzw. Wasser, welches mit Alkylpersulfonsäure und Schwefeldioxid zu Schwefelsäure reagiert. Um die Ausbeute an den beiden Endprodukten Alkylsulfonsäure und Schwefelsäure möglichst hoch zu halten und Nebenreaktionen zu unterdrücken, wird diese Reaktion üblicherweise nur bis zu Umsetzungsgraden von 1 % durchgeführt und danach abgebrochen.

Olefinsulfonate werden technisch durch Reaktion von α-Olefinen mit Schwefeltrioxid hergestellt. Hierbei bilden sich intermediär Zwitterionen, welche sich zu sogenannten Sultonen cyclisieren. Unter geeigneten Bedingungen (alkalische oder saure Hydrolyse) reagieren diese Sultone zu Hydroxylalkansulfonsäuren bzw. Alkensulfonsäuren, welche beide ebenfalls als Aniontensidsäuren eingesetzt werden können.

Alkylbenzolsulfonate als leistungsstarke anionische Tenside sind seit den dreißiger Jahren des letzten Jahrhunderts bekannt. Damals wurden durch Monochlorierung von Kogasin-Fraktionen und anschließende Friedel-Crafts-Alkylierung Alkylbenzole hergestellt, die mit Oleum sulfoniert und mit Natronlauge neutralisiert wurden. Anfang der fünfziger Jahre wurde zur Herstellung von Alkylbenzolsulfonaten Propylen zu verzweigtem α-Dodecylen tetramerisiert und das Produkt über eine Friedel-Crafts-Reaktion unter Verwendung von Aluminiumtrichlorid oder Fluorwasserstoff zum Tetrapropylenbenzol umgesetzt, das nachfolgend sulfoniert und neutralisiert wurde. Diese ökonomische Möglichkeit der Herstellung von Tetrapropylenbenzolsulfonaten (TPS) führte zum Durchbruch dieser Tensidklasse, die nachfolgend die Seifen als Haupttensid in Wasch- und Reinigungsmitteln verdrängte.

Aufgrund der mangelnden biologischen Abbaubarkeit von TPS bestand die Notwendigkeit, neue Alkylbenzolsulfonate darzustellen, die sich durch ein verbessertes ökologische Verhalten auszeichnen. Diese Erfordernisse werden von linearen Alkylbenzolsulfonaten erfüllt, welche heute die fast ausschließlich hergestellten Alkylbenzolsulfonate sind und mit dem Kurzzeichen ABS bzw. LAS belegt werden.

Lineare Alkylbenzolsulfonate werden aus linearen Alkylbenzolen hergestellt, welche wiederum aus linearen Olefinen zugänglich sind. Hierzu werden großtechnisch Petroleumfraktionen mit Molekularsieben in die n-Paraffine der gewünschten Reinheit aufgetrennt und zu den n-Olefinen dehydriert, wobei sowohl α- als auch i-Olefine resultieren. Die entstandenen Olefine werden dann in Gegenwart saurer Katalysatoren mit Benzol zu den Alkylbenzolen umgesetzt, wobei die Wahl des Friedel-Crafts-Katalysators einen Einfluß auf die Isomerenverteilung der entstehenden linearen Alkylbenzole hat: Bei Verwendung von Aluminiumtrichlorid liegt der Gehalt der 2-Phenyl-Isomere in der Mischung mit den 3-, 4-, 5- und anderen Isomeren bei ca. 30 Gew.-%, wird hingegen Fluorwasserstoff als Katalysator eingesetzt, läßt sich der Gehalt an 2-Phenyl-Isomer auf ca. 20 Gew.-% senken. Die Sulfonierung der linearen Alkylbenzole schließlich gelingt heute großtechnisch mit Oleum, Schwefelsäure oder gasförmigem Schwefeltrioxid, wobei letzteres die weitaus größte Bedeutung hat. Zur Sulfonierung werden spezielle Film- oder Rohrbündelreaktoren eingesetzt, die als Produkt eine 97 Gew.-%ige Alkylbenzolsulfonsäure (ABSS) liefern, die im Rahmen der vorliegenden Erfindung als Aniontensidsäure einsetzbar ist.

Durch Wahl des Neutralisationsmittels lassen sich aus den ABSS die unterschiedlichsten Salze, d.h. Alkylbenzolsulfonate, gewinnen. Aus Gründen der Ökonomie ist es hierbei bevorzugt, die Alkalimetallsalze und unter diesen bevorzugt die Natriumsalze der ABSS herzustellen und einzusetzen. Diese lassen sich durch die allgemeine Formel IX beschreiben: 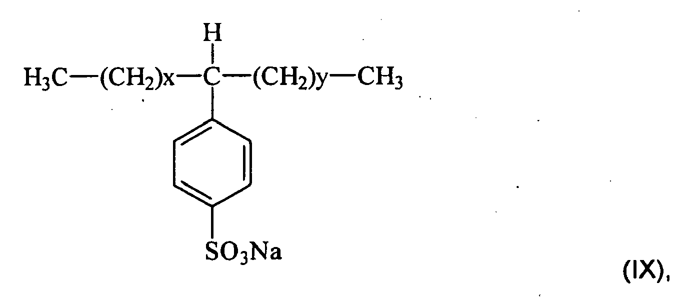 in der die Summe aus x und y üblicherweise zwischen 5 und 13 liegt. Erfindungsgemäß bevorzugt als Aniontensid in Säureform sind C₈₋₁₆-, vorzugsweise C₉₋₁₃-Alkylbenzolsulfonsäuren. Es ist im Rahmen der vorliegenden Erfindung weiterhin bevorzugt, C₈₋₁₆-, vorzugsweise C₉₋₁₃-Alkybenzolsulfonsäuren einzusetzen, die sich von Alkylbenzolen ableiten, welche einen Tetralingehalt unter 5 Gew.-%, bezogen auf das Alkylbenzol, aufweisen. Weiterhin bevorzugt ist es, Alkylbenzolsulfonsäuren zu verwenden, deren Alkylbenzole nach dem HF-Verfahren hergestellt wurden, so daß die eingesetzten C₈₋₁₆-, vorzugsweise C₉₋₁₃-Alkybenzolsulfonsäuren einen Gehalt an 2-Phenyl-Isomer unter 22 Gew.-%, bezogen auf die Alkylbenzolsulfonsäure, aufweisen.

Die vorstehend genannten Aniontenside in ihrer Säureform können alleine oder in Mischung miteinander eingesetzt werden. Es ist aber auch möglich und bevorzugt, daß dem Aniontensid in Säureform vor der Zugabe auf das/die Trägermaterial(ien) weitere, vorzugsweise saure, Inhaltsstoffe von Wasch- und Reinigungsmitteln in Mengen von 0,1 bis 40 Gew.-%, vorzugsweise von 1 bis 15 Gew.-% und insbesondere von 2 bis 10 Gew.-%, jeweils bezogen auf das Gewicht der umzusetzenden Mischung, zugemischt werden.

Selbstverständlich ist es auch möglich, die Aniontenside teil- oder vollneutralisiert einzusetzen. Diese Salze können dann als Lösung, Suspension oder Emulsion in der Granulierflüssigkeit vorliegen, aber auch als Feststoff Bestandteil des Feststoffbetts sein. Als Kationen für solche Aniontenside bieten sich neben den Alkalimetallen (hier insbesondere nach Anspruch- und K-Salze) Ammoniumsowie Mono-, Di- oder Triethanolalkonium-Ionen an. Anstelle von Mono-, Di- oder Triethanolamin können auch die analogen Vertreter des Mono-, Di- oder Trimethanolamins bzw. solche der Alkanolamine höherer Alkohole quaterniert und als Kation zugegen sein.

Auch Kationtenside lassen sich mit Vorteil als Aktivsubstanz einsetzen. Das Kationtensid kann dabei in seiner Lieferform direkt in den Mischer gegeben werden, oder in Form einer flüssigen bis pastösen Kationtensid-Zubereitungsform auf den festen Träger aufgedüst werden. Solche Kationtensid-Zubereitungsformen lassen sich beispielsweise durch Mischen handelsüblicher Kationtenside mit Hilfsstoffen wie nichtionischen Tensiden, Polyethylenglycolen oder Polyolen herstellen. Auch niedere Alkohole wie Ethanol und Isopropanol können eingesetzt werden, wobei die Menge an solchen niederen Alkoholen in der flüssigen Kationtensid-Zubereitungsform aus den obengenannten Gründen unter 10 Gew.-% liegen sollte.

Als Kationtenside kommen für die erfindungsgemäßen Mittel alle üblichen Stoffe in Betracht, wobei Kationtenside mit textilweichmachender Wirkung deutlich bevorzugt sind.

Die erfindungsgemäßen Mittel können als kationische Aktivsubstanzen mit textilweichmachender Wirkung ein oder mehrere kationische, textilweichmachende Mittel der Formeln X, XI oder XII enthalten: 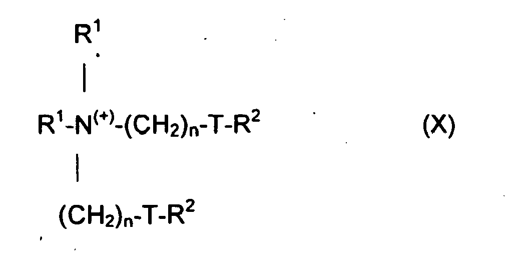 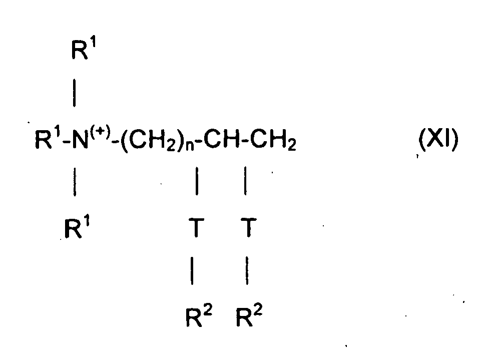 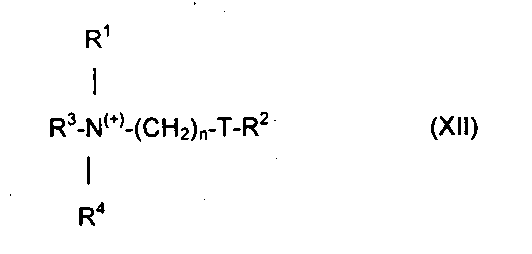 worin jede Gruppe R¹ unabhängig voneinander ausgewählt ist aus C₁₋₆-Alkyl-, - Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist.

In bevorzugten Ausführungsformen der vorliegenden Erfindung enthalten die Mittel zusätzlich Niotensid(e) als Aktivsubstanz.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Außerdem können als weitere nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel XIII, 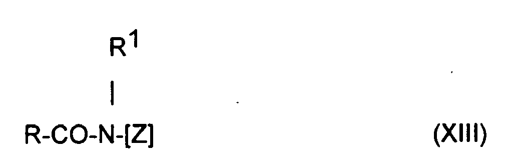 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel XIV, 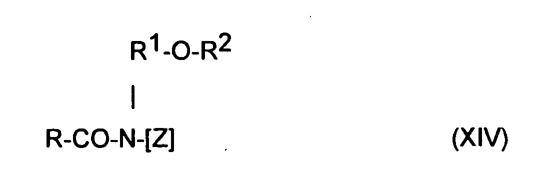 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Es ist für viele Anwendungen besonders bevorzugt, wenn das Verhältnis von Aniontensid(en) zu Niotensid(en) zwischen 10:1 und 1:10, vorzugsweise zwischen 7,5:1 und 1:5 und insbesondere zwischen 5:1 und 1:2 beträgt. Bevorzugt sind dabei erfindungsgemäße Behälter, die Tensid(e), vorzugsweise anionische(s) und/oder nichtionische(s) Tensid(e), in Mengen von 5 bis 80 Gew.-%, vorzugsweise von 7,5 bis 70 Gew.-%, besonders bevorzugt von 10 bis 60 Gew.-% uns insbesondere von 12,5 bis 50 Gew.-%, jeweils bezogen auf das Gewicht der umschlossenen Feststoffe, enthalten.

Wie bereits erwähnt, beschränkt sich der Einsatz von Tensiden bei Reinigungsmitteln für das maschinelle Geschirrspülen vorzugsweise auf den Einsatz nichtionischer Tenside in geringen Mengen. Erfindungsgemäße Mittel für das maschinelle Geschirrspülen enthalten daher vorzugsweise nur bestimmte nichtionische Tenside, die nachstehend beschrieben sind. Als Tenside werden in maschinellen Geschirrspülmitteln üblicherweise lediglich schwachschäumende nichtionische Tenside eingesetzt. Vertreter aus den Gruppen der anionischen, kationischen oder amphoteren Tenside haben dagegen eine geringere Bedeutung. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Insbesondere bei Reinigungsmitteln für das maschinelle Geschirrspülen ist es bevorzugt, daß diese ein nichtionisches Tensid enthalten, das einen Schmelzpunkt oberhalb Raumtemperatur aufweist, bevorzugt ein nichtionisches Tensid mit einem Schmelzpunkt oberhalb von 20°C. Bevorzugt einzusetzende nichtionische Tenside weisen Schmelzpunkte oberhalb von 25°C auf, besonders bevorzugt einzusetzende nichtionische Tenside haben Schmelzpunkte zwischen 25 und 60°C, insbesondere zwischen 26,6 und 43,3°C.

Geeignete nichtionische Tenside, die Schmelz- bzw. Erweichungspunkte im genannten Temperaturbereich aufweisen, sind beispielsweise schwachschäumende nichtionische Tenside, die bei Raumtemperatur fest oder hochviskos sein können. Werden bei Raumtemperatur hochviskose Niotenside eingesetzt, so ist bevorzugt, daß diese eine Viskosität oberhalb von 20 Pas, vorzugsweise oberhalb von 35 Pas und insbesondere oberhalb 40 Pas aufweisen. Auch Niotenside, die bei Raumtemperatur wachsartige Konsistenz besitzen, sind bevorzugt.

Bevorzugt als bei Raumtemperatur feste einzusetzende Niotenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen (PO/EO/PO)-Tenside. Solche (PO/EO/PO)-Niotenside zeichnen sich darüber hinaus durch gute Schaumkontrolle aus.

In einer bevorzugten Ausführungsform der vorliegenden Erfindung ist das nichtionische Tensid mit einem Schmelzpunkt oberhalb Raumtemperatur ein ethoxyliertes Niotensid, das aus der Reaktion von einem Monohyoroxyalkanol oder Alkylphenol mit 6 bis 20 C-Atomen mit vorzugsweise mindestens 12 Mol, besonders bevorzugt mindestens 15 Mol, insbesondere mindestens 20 Mol Ethylenoxid pro Mol Alkohol bzw. Alkylphenol hervorgegangen ist.

Ein besonders bevorzugtes bei Raumtemperatur festes, einzusetzendes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12 Mol, vorzugsweise mindestens 15 Mol und insbesondere mindestens 20 Mol Ethylenoxid gewonnen. Hierunter sind die sogenannten "narrow range ethoxylates" (siehe oben) besonders bevorzugt.

Das bei Raumtemperatur feste Niotensid besitzt vorzugsweise zusätzlich Propylenoxideinheiten im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- bzw. Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus.

Weitere besonders bevorzugt einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan.

Nichtionische Tenside, die mit besonderem Vorzug eingesetzt werden können, sind beispielsweise unter dem Namen Poly Tergent^{®} SLF-18 von der Firma Olin Chemicals erhältlich.
1 Ein weiter bevorzugtes Tensid läßt sich durch die Formel

R¹O[CH₂CH(CH₃)O]ₓ[CH₂CH₂O]_{y}[CH₂CH(OH)R²]

beschreiben, in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 und y für einen Wert von mindestens 15 steht.

Weitere bevorzugt einsetzbare Niotenside sind die endgruppenverschlossenen Poly(oxyalkylierten) Niotenside der Formel

R¹O[CH₂CH(R³)O]ₓ[CH₂]ₖCH(OH)[CH₂]ⱼOR²

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, isoPropyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder - CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Insbesondere bevorzugte endgruppenverschlossenen Poly(oxyalkylierte) Alkohole der obenstehenden Formel weisen Werte von k = 1 und j = 1 auf, so daß sich die vorstehende Formel zu

R¹O[CH₂CH(R³)O]ₓCH₂CH(OH)CH₂OR²

vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

### Enzyme

Erfindungsgemäße Mittel können zur Steigerung der Wasch-, beziehungsweise Reinigungsleistung Enzyme enthalten, wobei prinzipiell alle im Stand der Technik für diese Zwecke etablierten Enzyme einsetzbar sind. Hierzu gehören insbesondere Proteasen, Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von 1 x 10⁻⁶ bis 5 Gewichts-Prozent bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsværd, Dänemark, erhältlich. Die Subtilisine 147 und 309 werden unter den Handelsnamen Esperase^{®}, beziehungsweise Savinase^{®} von der Firma Novozymes vertrieben. Von der Protease aus *Bacillus lentus* DSM 5483 leiten sich die unter der Bezeichnung BLAP^{®} geführten Varianten ab.

Weitere brauchbare Proteasen sind beispielsweise die unter den Handelsnamen Durazym^{®}, Relase^{®}, Everlase^{®}, Nafizym, Natalase^{®}, Kannase^{®} und Ovozymes^{®} von der Firma Novozymes, die unter den Handelsnamen, Purafect^{®}, Purafect^{®} OxP und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß einsetzbare Amylasen sind die α-Amylasen aus *Bacillus licheniformis,* aus *B. amyloliquefaciens* oder aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Des weiteren sind für diesen Zweck die α-Amylase aus *Bacillus sp.* A 7-7 (DSM 12368) und die Cyclodextrin-Glucanotransferase (CGTase) aus *B. agaradherens* (DSM 9948) hervorzuheben; ebenso sind Fusionsprodukte der genannten Moleküle einsetzbar.

Darüber hinaus sind die unter den Handelsnamen Fungamyl^{®} von der Firma Novozymes erhältlichen Weiterentwicklungen der α-Amylase aus *Aspergillus niger* und *A. oryzae* geeignet. Ein weiteres Handelsprodukt ist beispielsweise die Amylase-LT^{®}.

Erfindungsgemäße Mittel können Lipasen oder Cutinasen insbesondere wegen ihrer Triglycerid-spaltenden Aktivitäten enthalten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus *Fusarium solani pisi* und *Humicola insolens* isoliert worden sind. Ebenso brauchbare Lipasen sind von der Firma Amano unter den Bezeichnungen Lipase CE^{®}, Lipase P^{®}, Lipase B^{®}, beziehungsweise Lipase CES^{®}, Lipase AKG^{®}, Bacillis sp. Lipase^{®}, Lipase AP^{®}, Lipase M-AP^{®} und Lipase AML^{®} erhältlich. Von der Firma Genencor sind beispielsweise die Lipasen beziehungsweise Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Erfindungsgemäße Mittel können, insbesondere wenn sie für die Behandlung von Textilien gedacht sind, Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen werden von der Firma Novozymes unter dem Handelsnamen Celluzyme^{®} angeboten. Die ebenfalls von der Firma Novozymes erhältlichen Produkte Endolase^{®} und Carezyme^{®} basieren auf der 50 kD-EG, beziehungsweise der 43 kD-EG aus *H. insolens* DSM 1800. Weitere mögliche Handelsprodukte dieser Firma sind Cellusoft^{®} und Renozyme^{®}. Ebenso ist die 20 kD-EG Cellulase aus *Melanocarpus*, die von der Firma AB Enzymes, Finnland, unter den Handelsnamen Ecostone^{®} und Biotouch^{®} erhältlich ist, einsetzbar. Weitere Handelprodukte der Firma AB Enzymes sind Econase^{®} und Ecopulp^{®}. Eine weitere geeignete Cellulase aus *Bacillus sp.* CBS 670.93 ist von der Firma Genencor unter dem Handelsnamen Puradax^{®} erhältlich. Weitere Handelsprodukte der Firma Genencor sind "Genencor detergent cellulase L" und IndiAge^{®}Neutra.

Erfindungsgemäße Mittel können weitere Enzyme enthalten, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Geeignete Mannanasen sind beispielsweise unter den Namen Gamanase^{®} und Pektinex AR^{®} von der Firma Novozymes, unter dem Namen Rohapec^{®} B1L von der Firma AB Enzymes und unter dem Namen Pyrolase^{®} von der Firma Diversa Corp., San Diego, CA, USA erhältlich. Die aus *B. subtilis* gewonnene β-Glucanase ist unter dem Namen Cereflo^{®} von der Firma Novozymes erhältlich.

Zur Erhöhung der bleichenden Wirkung können erfindungsgemäße Wasch- oder Reinigungsmittel Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) enthalten. Als geeignete Handelsprodukte sind Denilite^{®} 1 und 2 der Firma Novozymes zu nennen. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die in erfindungsgemäßen Mitteln eingesetzten Enzyme stammen entweder ursprünglich aus Mikroorganismen, etwa der Gattungen *Bacillus, Streptomyces, Humicola*, oder *Pseudomonas*, und/oder werden nach an sich bekannten biotechnologischen Verfahren durch geeignete Mikroorganismen produziert, etwa durch transgene Expressionswirte der Gattungen *Bacillus* oder filamentöse Fungi.

Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Erfindungsgemäßen Mitteln können die Enzyme in jeder nach dem Stand der Technik etablierten Form zugesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so daß ein einzelnes Granulat mehrere Enzymaktivitäten aufweist.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Erfindungsgemäße Mittel können zu diesem Zweck Stabilisatoren enthalten; die Bereitstellung derartiger Mittel stellt eine bevorzugte Ausführungsform der vorliegenden Erfindung dar.

Eine Gruppe von Stabilisatoren sind reversible Proteaseinhibitoren. Häufig werden Benzamidin-Hydrochlorid, Borax, Borsäuren, Boronsäuren oder deren Salze oder Ester verwendet, darunter vor allem Derivate mit aromatischen Gruppen, etwa ortho-,meta- oder para-substituierte Phenylboronsäuren, beziehungsweise deren Salze oder Ester. Weiterhin sind Peptidaldehyde, das heißt Oligopeptide mit reduziertem C-Terminus geeignet. Als peptidische Proteaseinhibitoren sind unter anderem Ovomucoid und Leupeptin zu erwähnen; eine zusätzliche Option ist die Bildung von Fusionsproteinen aus Proteasen und Peptid-Inhibitoren.

Weitere Enzymstabilisatoren sind Aminoalkohole wie Mono-, Di-, Triethanol- und -Propanolamin und deren Mischungen, aliphatische Carbonsäuren bis zu C₁₂, wie Bernsteinsäure, andere Dicarbonsäuren oder Salze der genannten Säuren. Auch endgruppenverschlossene Fettsäureamidalkoxylate sind als Stabilisatoren einsetzbar.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Weiterhin schützt auch Di-Glycerinphosphat gegen Denaturierung durch physikalische Einflüsse. Ebenso werden Calciumsalze verwendet, wie beispielsweise Calciumacetat oder Calcium-Formiat sowie Magnesiumsalze.

Polyamid-Oligomere oder polymere Verbindungen wie Lignin, wasserlösliche Vinyl-Copolymere oder, wie Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind die linearen C₈-C₁₈ Polyoxyalkylene. Alkylpolyglycoside können gemäß den ebenfalls die enzymatischen Komponenten des erfindungsgemäßen Mittels stabilisieren und sogar in ihrer Leistung steigern. Vernetzte N-haltige Verbindungen erfüllen eine Doppelfunktion als Soil-release-Agentien und als Enzym-Stabilisatoren.

Reduktionsmittel und Antioxidantien wie Natrium-Sulfit oder reduzierende Zucker erhöhen die Stabilität der Enzyme gegenüber oxidativem Zerfall.

Bevorzugt werden Kombinationen von Stabilisatoren verwendet, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren kann durch die Kombination mit Borsäure und/oder Borsäurederivaten und Polyolen gesteigert und gemäß durch die zusätzliche Verwendung von zweiwertigen Kationen, wie zum Beispiel Calcium-lonen weiter verstärkt werden.

Besonders bevorzugt ist im Rahmen der vorliegenden Erfindung der Einsatz flüssiger Enzymformulierungen. Hier sind erfindungsgemäße Mittel bevorzugt, die zusätzlich Enzyme und/oder Enzymzubereitungen, vorzugsweise feste und/oder flüssige Protease-Zubereitungen und/oder Amylase-Zubereitungen, in Mengen von 1 bis 5 Gew.-%, vorzugsweise von 1,5 bis 4,5 und insbesondere von 2 bis 4 Gew.-%, jeweils bezogen auf das gesamte Mittel, enthalten.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den erfindungsgemäßen Granulaten bevorzugt.

### Bezugszeichen:

- 1: Verschluss
- 2: Dosiervolumen
- 3: Kammer
- 4: Verschlussmittel, Schraubgewinde
- 5: Mantelfläche
- 6: Boden
- 7: Mantelfläche
- 8: Boden
- 9: Kammerdecke
- 10: Öffnung
- 11: Verschlussplatte
- 12: Becherelement
- 13: Kragen
- 14: Nut
- 15: Nase
- 16: Verschlusskappe
- 17: Überwurfring

Weitere Vorteile der Erfindung werden anhand eines in der Zeichnung dargestellten Ausführungsbeispiels erläutert. Es zeigen:
- Figur 1:: Dosierverschluss mit Kammer im Querschnitt
- Figur 2:: Dosierverschluss ohne Kammer im Querschnitt
- Figur 3:: Becherelement der Kammer im Querschnitt
- Figur 4:: Verschlussplatte der Kammer im Querschnitt

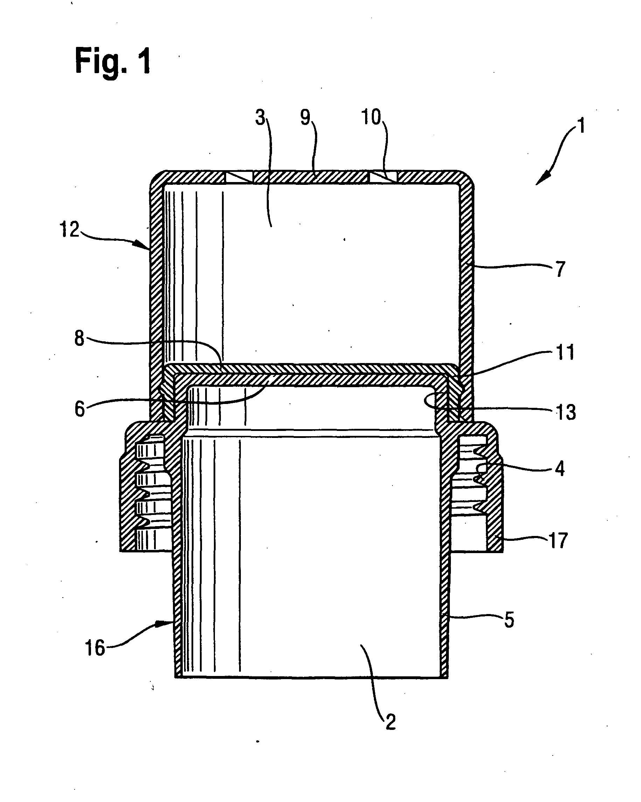

Figur 1 zeigt den erfindungsgemäßen Dosierverschluss 1 mit der Kammer 3 zur Aufnahme von Aktivsubstanzen (nicht dargestellt) im Querschnitt.

Der Dosierverschluss 1 ist zweiteilig aufgebaut und weist eine Kammer 3 und eine Verschlusskappe 16 auf, die lösbar miteinander verbunden sind.

Die Verschlusskappe 16 weist ein becherartiges Dosiervolumen 2 auf, welches durch die im wesentlichen zylinderförmige Mantelfläche 5 und den Boden 6 der Verschlusskappe 16 gebildet wird.

An der Mantelfläche 5 ist ein Überwurfring 17 angeordnet, der ein innenliegendes Schraubgewinde 4 aufweist und derart ausgestaltet ist, dass beim Zusammenwirken mit der Behälteröffnung in der Verschlussstellung des Verschlusses 1 ein Austritt von Produkt aus dem Behälter verhindert wird.

Der Überwurfring 17 und die Mantelfläche 5 sind einteilig ausgeformt wobei der Überwurfring 17 derart an der Mantelfläche positioniert ist, dass zwischen dem Boden 6 und der Schulter des Überwurfringes 17 ein Kragen 13 ausgebildet wird. Der Kragen 13 steht im wesentlichen senkrecht auf der Schulter des Überwurfrings 17 und zum Boden 6 der Verschlusskappe 16, wodurch eine domartige Erhöhung ausgebildet ist, die aus der Verschlusskappe 16 herausragt.

Die Kammer 3 zur Aufnahme von Aktivsubstanzen ist zweiteilig ausgeführt. Die Kammer 3 besteht aus einem becherförmigen Element 3, dass durch den im wesentlichen zylindrischen Kammermantel 7 und den Kammerkopf 9 gebildet wird, sowie aus einer im Querschnitt u-förmigen Verschlussplatte 11, die im zusammengebauten Zustand den Boden 8 der Kammer 3 ausbildet und das Becherelement 12 verschließt.

Die Verbindung zwischen der Verschlussplatte 11 und dem Becherelement 12 kann als Klebverbindung und/oder Pressverbindung und/oder Rastverbindung ausgeführt sein.

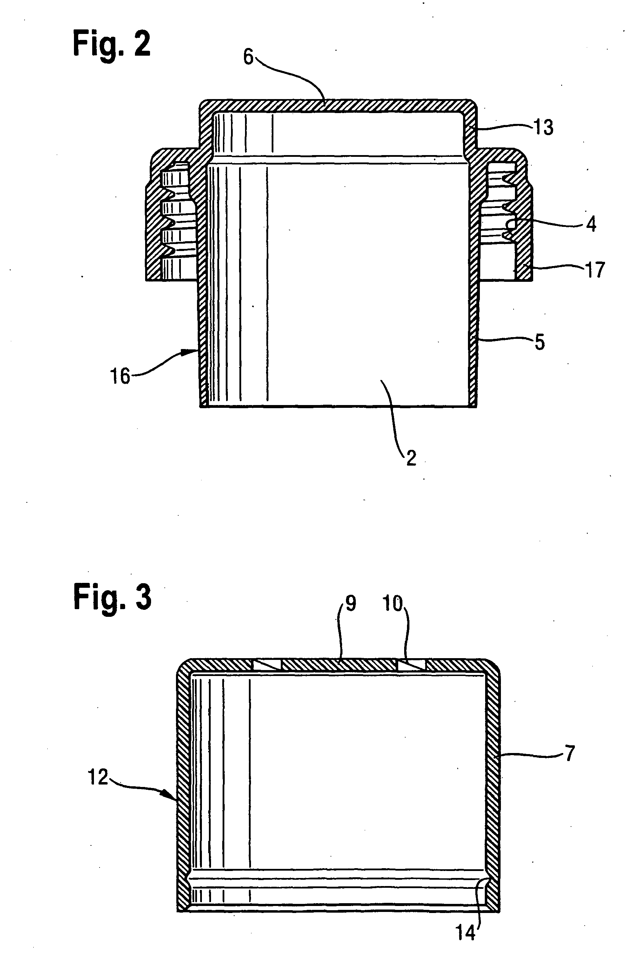

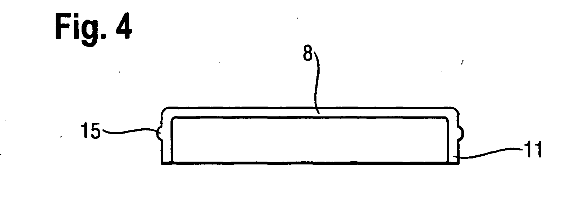

Wie in Figur 3 dargestellt, weist die Mantelfläche 7 des Becherelements 12 eine umlaufende Nut 14 auf, die zur Aufnahme der, wie in Figur 4 gezeigt, an der Verschlussplatte 11 ausgeformten umlaufenden Nase 15 dient, wodurch das Becherelement 12 und die Verschlussplatte 11 kraft- und formschlüssig miteinander verbunden sind.

Die Kammer 3 weist eine im Querschnitt u-förmige Vertiefung auf, die derart ausgestaltet ist, dass die Kammer 3 mittels Presspassung auf der domartigen Erhöhung der Verschlusskappe 16 lösbar fixiert ist.

Die Presspassung zwischen der Kammer 3 und der Verschlusskappe 16 ist dabei derart dimensioniert, dass sich durch die Abnahme der Kammer 3 von der Verschlusskappe 16 die Verbindung zwischen der Verschlusskappe 16 und der Kammer 3 löst ohne dass das Becherelement 12 von der Verschlussplatte 11 abgetrennt wird.

Der Verschluss 1 ist vorzugsweise aus Kunststoff gefertigt. Die Verschlusskappe 16 und die Kammer 3 können aus gleichen oder unterschiedlichen Kunststoffen gefertigt sein. Besonders vorteilhaft ist es, die Verschlusskappe 16 und die Kammer 3 aus einem oder mehreren Polyolefin-Kunststoffen, wie beispielsweise Polyethylen oder Polypropylen, herzustellen.

## Patentansprüche

1. Verschluss (1) zum Verschließen der Öffnung eines Behälters für insbesondere fließ- oder schüttfähige Produkte, umfassend eine Verschlusskappe (16), ein Mittel (4) zum im wesentlichen dichten Verschließen der Öffnung eines Behälters, wobei das Verschlussmittel (4) der Verschlusskappe (16) derart ausgestaltet ist, dass beim Zusammenwirken mit der Behälteröffnung in der Verschlussstellung des Verschlusses (1) ein Austritt von Produkt aus dem Behälter verhindert wird, sowie eine Kammer (3), die durch einen Kammerboden (8), einen Kammermantel (7) und eine Kammerdecke (9) gebildet ist und die zumindest teilweise mit mindestens einer Aktivsubstanz befüllt ist, wobei die Kammer (3) mindestens eine Öffnung (10) aufweist, die eine Abgabe einer Aktivsubstanz aus dem Inneren der Kammer (3) an die Umgebung erlaubt und die Kammer (3) gegenüber der Verschlusskappe (16) dicht ausgebildet ist **dadurch gekennzeichnet, dass** die Kammer (3) lösbar an der Verschlusskappe (16) fixierbar ist.

2. Verschluss nach Anspruch 1, **dadurch gekennzeichnet, dass** die Kammer (3) form- und/oder stoff- und/oder kraftschlüssig am Verschluss (1) angeordnet ist.

3. Verschluss nach einem der vorherigen Ansprüche, **dadurch gekennzeichnet, dass** die Kammer (3) durch eine Zugkraft von größer als 1N entlang der Längsachse von der Verschlusskappe (16) lösbar ist.

4. Verschluss nach einem der vorherigen Ansprüche, **dadurch gekennzeichnet, dass** die Verschlusskappe (16) ein durch eine Mantelfläche (5) und einen Boden (6) becherartig ausgeformtes Dosiervolumen (2) aufweist.

5. Verschluss nach einem der vorherigen Ansprüche, **dadurch gekennzeichnet, dass** die Kammer (3) zweiteilig aus einem becherförmigen Element (12) und einer Verschlussplatte (11), durch welche die Öffnung des becherförmigen Elements (12) stoff- und/oder form- und/oder kraftschlüssig verschließbar ist, gebildet ist.

6. Verschluss Anspruch 5, **dadurch gekennzeichnet, dass** das becherförmige Element (12) auf der Mantelfläche (7) eine umlaufende Nut (14) aufweist, die mit einer korrespondierenden Nase (15) der Verschlussplatte (11) derart zusammenwirkt, dass eine Rastverbindung zwischen Verschlussplatte (11) und Becherelement (12) ausbildbar ist.

7. Verschluss nach einem der vorherigen Ansprüche 5 oder 6, **dadurch gekennzeichnet, dass** die Öffnung des becherförmigen Elements (12) durch die Verschlussplatte (11) im Wesentlichen dicht verschließbar ist.

8. Verschluss nach einem der vorherigen Ansprüche 5 bis 7, **dadurch gekennzeichnet, dass** die Verschlussplatte (11) im Querschnitt u-förmig ausgeformt ist.

9. Verschluss nach einem der vorherigen Ansprüche, **dadurch gekennzeichnet, dass** das Mittel (4) zum Verschließen der Ausgussöffnung als Überwurfring mit einem innen liegenden Schraubgewinde ausgebildet ist.

10. Verschluss nach einem der vorherigen Ansprüche 4 bis 9, **dadurch gekennzeichnet, dass** der Dosierverschluss einen Kragen (13) aufweist, welcher eine aus der Verschlusskappe (16) hinausragende domartige Erhöhung ausbildet, auf der die Kammer (3), vorzugsweise durch aufstecken, fixierbar ist.

11. Verschluss nach einem der vorherigen Ansprüche, **dadurch gekennzeichnet, dass** die Kammer (3) außenseitig ein Befestigungselement, vorzugsweise ein versiegelter Klebestreifen, zur Fixierung der Kammer (3) an Strukturen außerhalb des Dosierverschlusses (1) aufweist.

12. Verschluss nach einem der vorherigen Ansprüche, **dadurch gekennzeichnet, dass** mindestens eine Aktivsubstanz aus der Gruppe der Duftstoffe, Bleichmittel, Reinigungssubstanzen, Lösemittel, Tenside, Farbstoffe, Enzyme, hygroskopische Substanzen, Flammhemmer, Härter, Verlaufsmittel, Netzmittel, Dispergiermittel, Schaumbildner, Entschäumer, Wachse, Silikone, Entlüfter, Korrosionsschutzmittel, Biozide, Insektizide, Wasserenthärter, Konservierungsmittel, Adsorptionsmittel, Absorptionsmittel, Abrasivstoffe, Emulgatoren, Stabilisatoren, Vitamine, Mineralien, Substanzen oder Substanzgemische zur Hydrophilisierung von Oberfläche und/oder Indikatorsubstanzen, insbesondere zur Bestimmung der Wasserhärte, Keimbelastung, Temperatur und/oder Feuchtigkeit ausgewählt ist.

13. Verschluss nach einem der vorherigen Ansprüche, **dadurch gekennzeichnet, dass** die Aktivsubstanz ein mit Duftstoff beladenes Polymermaterial ist.

14. Verschluss nach Anspruch 13, **dadurch gekennzeichnet ,dass** das polymere Trägermaterial einen Schmelz- oder Erweichungspunkt zwischen 30 und 150°C, vorzugsweise zwischen 40 und 125°C, besonders vorzugsweise zwischen 60 und 100°C, ganz besonders bevorzugt von 70 bis 90°C und insbesondere zwischen 75 und 80°C, aufweist.

15. Verschluss nach einem der Ansprüche 13 bis 14 , **dadurch gekennzeichnet, dass** das polymere Trägermaterial mindestens eine Substanz aus der Gruppe umfassend EthylenNinylacetat- Copolymere, Polyethylen niederer oder hoher Dichte (LDPE, HDPE) oder Gemische derselben, Polypropylen, Polyethylen/Polypropylen-Copolymere, Polyether/Polyamid-Blockcopolymere, Styrol/Butadien-(Block-)Copolymere, Styrol/Isopren-(Block-)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat, Polyurethane, Polyvinylalkohole enthält.

16. Verschluss nach einem der Ansprüche 13 bis 15, **dadurch gekennzeichnet, dass** die Partikel einen mittleren Durchmesser von 0,5 bis 20 mm, vorzugsweise von 1 bis 10 mm und insbesondere von 3 bis 6 mm aufweisen.

17. Verschluss nach einem der Ansprüche 13 bis 16, **dadurch gekennzeichnet, dass** der Gewichtsanteil des bzw. der Duftstoffe 1 bis 70 Gew.-%, vorzugsweise 10 bis 60 Gew.-%, besonders bevorzugt 20 bis 50 Gew.-%, insbesondere 30 bis 40 Gew.-%, beträgt.

18. Verschluss nach einem der vorherigen Ansprüche, **dadurch gekennzeichnet**, der Verschluss (1) ein Verschlusselement für die Öffnungen (10) der Kammer (3) aufweist, welches in einer Verschlussposition die Abgabe von Aktivsubstanz aus der Kammer (3) an die Umgebung zumindest reduziert und in einer geöffneten Position die Abgabe von Aktivsubstanz aus der Kammer ermöglicht.

19. Verschluss nach Anspruch 18, **dadurch gekennzeichnet, dass** das Verschlusselement ein wiederholtes Freigeben und Verschließen der Öffnungen (10) ermöglicht.

20. Verpackung umfassend einen Behälter, insbesondere für fließ- oder schüttfähige Produkte, mit einer Öffnung zur Produktabgabe, **dadurch gekennzeichnet, dass**, die Öffnung mit einem Verschluss nach einem der vorherigen Ansprüche verschlossen ist.

## Claims

1. A closure (1) for closing the opening of a container for in particular flowable or pourable products, said closure (1) comprising a closure cap (16), a means (4) for closing the opening of a container in an essentially sealed manner, wherein said closure means (4) of the closure cap (16) is configured such that, upon co-operation with the container opening in the closed position of the closure (1), product is prevented from escaping from the container, and further comprising a chamber (3) that is formed by a chamber base (8), a chamber shell (7) and a chamber cover (9) and is filled, at least in part, with at least one active substance, wherein said chamber (3) has at least one opening (10) that allows an active substance to be discharged from the interior of the chamber (3) into the surroundings, and said chamber (3) is designed in a sealed manner in relation to the closure cap (16), **characterized in that** said chamber (3) can be fixed in a releasable manner on the closure cap (16).

2. The closure according to claim 1, **characterized in that** the chamber (3) is arranged on the closure (1) by positive interlocking and/or by material-locking and/or by force-fit.

3. The closure according to one of the preceding claims, **characterized in that** the chamber (3) can be detached by means of a tensile force of more than 1N along the longitudinal axis of the closure cap (16).

4. The closure according to one of the preceding claims, **characterized in that** the closure cap (16) exhibits a cup-like dosing volume (2) formed by a shell surface (5) and a base (6).

5. The closure according to one of the preceding claims, **characterized in that** the chamber (3) is formed in two parts of a cup-like element (12) and a closure plate (11), by which the opening of said cup-like element (12) can be closed by positive interlocking and/or by material-locking and/or by force-fit.

6. The closure according to claim 5, **characterized in that** the cup-like element (12) possesses a circumferential groove (14) on the shell surface (7), said groove (14) cooperating with a corresponding lug (15) of the closure plate (11) such that a snap-fit connection can be formed between closure plate (11) and cup-like element (12).

7. The closure according to one of the preceding claims 5 or 6, **characterized in that** the opening of the cup-like element (12) can be essentially tightly closed by the closure plate (11).

8. The closure according to one of the preceding claims 5 to 7, **characterized in that** the closure plate (11) has a u-shaped cross section.

9. The closure according to one of the preceding claims, **characterized in that** the means (4) for closing the pouring outlet is configured as a retaining ring having a screw thread on the inside.

10. The closure according to one of the preceding claims 4 to 9 **characterized in that** the dosing closure possesses a collar (13) that forms a dome-like elevation that protrudes out of the closure cap (16), onto which the chamber (3) can be fixed, preferably by snapping on.

11. The closure according to one of the preceding claims, **characterized in that** the chamber (3) possesses a fastening element on the outside, preferably a sealed adhesive strip, for fixing the chamber (3) to structures outside the dosing closure (1).

12. The closure according to one of the preceding claims, **characterized in that** at least one active substance is selected from the group of the fragrances, bleaching agents, cleaning substances, solvents, surfactants, dyes, enzymes, hygroscopic substances, flame retardants, hardeners, levelling agents, wetting agents, dispersants, foaming agents, defoamers, waxes, silicones, exhausters, corrosion protection compositions, biocides, insecticides, water softeners, preservatives, adsorbents, absorbents, abrasive materials, emulsifiers, stabilisers, vitamins, minerals, substances and/or mixtures of substances for rendering surfaces hydrophilic and/or indicators, particularly for determining the water hardness, the presence of germs, temperature and/or moisture.

13. The closure according to one of the preceding claims, **characterized in that** the active substance is a polymeric material loaded with fragrance.

14. The closure according to claim 13, **characterized in that** the polymeric carrier material has a melting point or softening point between 30 and 150 °C, preferably between 40 and 125 °C, particularly preferably between 60 and 100 °C, quite particularly preferably from 70 to 90 °C and especially between 75 and 80 °C.

15. The closure according to one of claims 13 to 14, **characterized in that** the polymeric carrier material comprises at least one substance from the group comprising ethylene/vinyl acetate copolymers, low density or high density polyethylene (LDPE, HDPE) or mixtures thereof, polypropylene, polyethylene/polypropylene copolymers, polyether/polyamide block copolymers, styrene/butadiene (block) copolymers, styrene/isoprene (block) copolymers, styrene/ethylene/butylene copolymers, acrylonitrile/butadiene/styrene copolymers, acrylonitrile/butadiene copolymers, polyether ester, polyisobutene, polyisoprene, ethylene/ethyl acrylate copolymers, polyamides, polycarbonate, polyester, polyacrylonitrile, polymethyl methacrylate, polyurethanes, polyvinyl alcohols.

16. The closure according to one of claims 13 to 15, **characterized in that** the mean diameter of the particles is from 0.5 to 20 mm, preferably from 1 to 10 mm and especially from 3 to 6 mm.

17. The closure according to one of claims 13 to 16, **characterized in that** the weight fraction of the fragrance(s) is 1 to 70 wt.%, preferably 10 to 60 wt.%, particularly preferably 20 to 50 wt.%, especially 30 to 40 wt.%.

18. The closure according to one of the preceding claims, **characterized in that** the closure (1) possesses a closure element for the opening (10) of the chamber (3), said closure element, in a closed position, enabling the discharge of active substance, from the chamber (3) into the surroundings, to be at least reduced, and, in an open position, enabling the discharge of active substance from the chamber.

19. The closure according to claim 18, **characterized in that** the closure element enables a repeated release and closing of the opening (10).

20. The closure comprising a container, in particular for flowable or pourable products, having an opening for discharging product, **characterized in that** the opening is closed with a closure according to one of the preceding claims.

## Revendications

1. Fermeture (1) destinée à fermer l'orifice d'un récipient, en particulier pour produits pouvant couler ou être déversés, comprenant un capuchon de fermeture (16), un moyen (4) destiné à fermer l'orifice d'un récipient de manière essentiellement étanche, ledit moyen de fermeture (4) du capuchon de fermeture (16) étant configuré de telle manière qu'en coopérant avec l'orifice du récipient en position fermée de la fermeture (1), il empêche du produit de sortir du récipient, ainsi qu'une chambre (3), laquelle est formée par un fond de chambre (8), une enveloppe de chambre (7) et une couverture de chambre (9) et laquelle est remplie, au moins partiellement, avec au moins une substance active, ladite chambre (3) comportant au moins un orifice (10), permettant de distribuer une substance active à l'environnement depuis l'intérieur de la chambre (3), et ladite chambre (3) étant réalisée de manière étanche vis-à-vis du capuchon de fermeture (16), **caractérisée en ce que** la chambre (3) peut être fixée de manière amovible sur le capuchon de fermeture (16).

2. Fermeture selon la revendication 1, **caractérisée en ce que** la chambre (3) est agencée sur la fermeture (1) par liaison de forme et/ou de matière et/ou de force.

3. Fermeture selon l'une des revendications précédentes, **caractérisée en ce que** la chambre (3) peut être détachée du capuchon de fermeture (16) par une force de traction supérieure à 1N le long de l'axe longitudinal.

4. Fermeture selon l'une des revendications précédentes, **caractérisée en ce que** le capuchon de fermeture (16) comporte un volume de dosage (2) de type pot, formé par une surface latérale (5) et un fond (6).

5. Fermeture selon l'une des revendications précédentes, **caractérisée en ce que** la chambre (3) est formée en deux parties par un élément (12) en forme de pot et une plaque d'obturation (11) permettant de fermer l'orifice de l'élément (12) en forme de pot par liaison de matière et/ou de forme et/ou de force.

6. Fermeture selon la revendication 5, **caractérisée en ce que** l'élément (12) en forme de pot comporte sur la surface de l'enveloppe (7) une rainure continue (14) coopérant avec un ergot (15) correspondant de la plaque d'obturation, de manière à permettre la formation d'une liaison d'encliquetage entre plaque d'obturation (11) et élément formant pot (12).

7. Fermeture selon l'une des revendications précédentes 5 ou 6, **caractérisée en ce que** l'orifice de l'élément (12) en forme de pot peut être fermé par la plaque d'obturation (11) de manière essentiellement étanche.

8. Fermeture selon l'une des revendications précédentes 5 à 7, **caractérisée en ce que** la plaque d'obturation (11) a une section en forme de U.

9. Fermeture selon l'une des revendications précédentes, **caractérisée en ce que** le moyen (4) destiné à fermer l'orifice verseur est conçu sous forme d'anneau à chapeau pourvu d'un filetage interne.

10. Fermeture selon l'une des revendications précédentes 4 à 9, **caractérisée en ce que** la fermeture de dosage comporte une collerette (13) formant un relief en forme de dôme, qui fait saillie du capuchon de fermeture (16) et sur lequel la chambre (3) peut être fixée, de préférence par emboîtement.

11. Fermeture selon l'une des revendications précédentes, **caractérisée en ce que** la chambre (3) comporte, côté extérieur, un élément de fixation, de préférence une bande adhésive scellée, destiné à fixer la chambre (3) sur des structures à l'extérieur de la fermeture de dosage (1).

12. Fermeture selon l'une des revendications précédentes, **caractérisée en ce qu'**au moins une substance active est choisie dans le groupe des odorants, agents de blanchiment, substances de nettoyage, solvants, agents tensioactifs, colorants, enzymes, substances hygroscopiques, agents ignifuges, durcisseurs, agents d'écoulement, agents mouillants, agents dispersants, agents moussants, agents antimoussants, cires, silicones, désaérateurs, agents anticorrosifs, biocides, insecticides, adoucisseurs d'eau, conservateurs, adsorbants, absorbants, abrasifs, émulsifiants, stabilisateurs, vitamines, minéraux, substances ou mélanges de substances d'hydrophilisation de surface et/ou substances indicatrices, en particulier pour déterminer la dureté de l'eau, la pollution par germes, la température et/ou l'humidité.

13. Fermeture selon l'une des revendications précédentes, **caractérisée en ce que** la substance active est un matériau polymère chargé en odorant.

14. Fermeture selon la revendication 13, **caractérisée en ce que** le support polymère a un point de fusion ou de ramollissement compris entre 30 et 150°C, de préférence entre 40 et 125°C, plus préférablement entre 60 et 100°C, encore plus préférablement entre 70 et 90°C et particulièrement entre 75 et 80°C.

15. Fermeture selon l'une des revendications 13 à 14, **caractérisée en ce que** le support polymère contient au moins une substance du groupe comprenant les copolymères éthylène-acétate de vinyle, le polyéthylène basse ou haute densité (LDPE, HDPE) ou des mélanges de ceux-ci, le polypropylène, les copolymères polyéthylène-polypropylène, les copolymères bloc polyéther-polyamide, les copolymères (bloc) styrène-butadiène, les copolymères (bloc) styrène-isoprène, les copolymères styrène-éthylène-butylène, les copolymères acrylonitrile-butadiène-styrène, les copolymères acrylonitrile-butadiène, le polyétherester, le polyisobutène, le polyisoprène, les copolymères éthylène-acrylate d'éthyle, les polyamides, le polycarbonate, le polyester, le polyacrylonitrile, le polyméthylméthacrylate, les polyuréthanes, les alcools polyvinyliques.

16. Fermeture selon l'une des revendications 13 à 15, **caractérisée en ce que** les particules ont un diamètre moyen compris entre 0,5 et 20 mm, de préférence entre 1 et 10 mm et plus particulièrement entre 3 et 6 mm.

17. Fermeture selon l'une des revendications 13 à 16, **caractérisée en ce que** la teneur pondérale de l'odorant, ou des odorants, est comprise entre 1 et 70 % en poids, de préférence entre 10 et 60 % en poids, plus préférablement entre 20 et 50 % en poids, particulièrement entre 30 et 40 % en poids.

18. Fermeture selon l'une des revendications précédentes, **caractérisée en ce que** la fermeture (1) comporte un élément de fermeture pour les orifices (10) de la chambre (3), lequel, dans une position de fermeture, réduit au moins la distribution à l'environnement de substance active en provenance de la chambre (3) et, dans une position ouverte, permet la distribution de substance active en provenance de ladite chambre.

19. Fermeture selon la revendication 18, **caractérisée en ce que** ledit élément de fermeture permet une ouverture et fermeture répétées des orifices (10).

20. Emballage comprenant un récipient, en particulier pour produits pouvant couler ou être déversés, avec un orifice de distribution de produit, **caractérisé en ce que** ledit orifice est fermé par une fermeture selon l'une des revendications précédentes.
